# FASCICULE DE BREVET EUROPEEN

(11) **EP 2 152 850 B1**
(45) Date de publication et mention de la délivrance du brevet: **01.04.2020**
(21) Numéro de dépôt: 08805948.0
(22) Date de dépôt: 06.06.2008
(51) Int. Cl.: C12M 1/12

(54) **DISPOSITIF DE LYSE DE MICROORGANISMES PRESENTS DANS UN ECHANTILLON ENVIRONNEMENTAL OU CLINIQUE ET D'EXTRACTION DES ACIDES NUCLEIQUES DESDITS MICROORGANISMES AUX FINS D'ANALYSE**
VORRICHTUNG ZUR LYSIS VON MIKROORGANISMEN AUS EINER UMWELT- ODER KRANKENHAUSPROBE, PROBE UND DIE ENTNAHME VON NUKLEINSÄUREN AUS DEN BESAGTEN MIKROORGANISMEN ZUR ANALYSE
DEVICE FOR THE LYSIS OF MICROORGANISMS PRESENT IN AN ENVIRONMENTAL OR CLINICAL SAMPLE AND THE EXTRACTION OF NUCLEIC ACIDS FROM SAID MICROORGANISMS FOR ANALYSIS

(30) Priorité: 07.06.2007 FR 0755540
(43) Date de publication de la demande: 17.02.2010
(73) Titulaire: BIOMERIEUX, 69280 Marcy-L'Etoile (FR)
(72) Inventeur: KREUWEL, Hermanus, Johannes, Maria, NL-5481 LW Schijndel (NL); VERWIMP, Emiel, Gerebern, Maria, B-2460 Kasterlee (BE)
(86) Numéro de dépôt international: PCT/FR2008/051012
(87) Numéro de publication internationale: WO 2009/001010

(56) Documents cités:
- WO-A-00/73412
- WO-A-2004/018704
- WO-A-2006/117676
- FR-A- 2 861 085
- US-A- 5 707 861
- US-A1- 2004 038 385
- US-A1- 2007 068 284

## Description

Le domaine technique de la présente invention est celui de l'analyse biologique. Plus particulièrement, la présente invention concerne un dispositif de lyse des microorganismes présents dans un échantillon environnemental, tel qu'un échantillon d'air, ou clinique.

On observe, depuis plusieurs années, une recrudescence des infections nosocomiales au sein des hôpitaux. Ces infections s'expliquent par la contamination des personnes hospitalisées, et donc par définition immunodéprimées, par des microorganismes pathogènes présents dans la sphère environnementale hospitalière et non détruits malgré le soin toujours important apporté à la désinfection des outils et des surfaces et au traitement de l'air. Eu égard à ces cas de plus en plus fréquents de contaminations microbiologiques environnementales, la mise au point de dispositifs et de méthodes permettant d'améliorer et de faciliter les contrôles environnementaux est devenu un enjeu majeur pour les professionnels de santé.

Au-delà du problème des infections nosocomiales, le contrôle des conditions environnementales est également devenu depuis plusieurs années, un souci récurrent dans le milieu industriel, en particulier les industries agro-alimentaires, les industries pharmaceutiques ou cosmétiques. Dans les industries agro-alimentaires, on sait les conséquences désastreuses sur la santé des consommateurs, que peut avoir la contamination des produits, voire de matières premières, par un microorganisme pathogène. En effet, les toxi-infections alimentaires, dues à des bactéries telles que celles du genre *Listeria* ou *Salmonella* sont aujourd'hui monnaie courante. Le contrôle de la qualité de l'air est également un processus-clé dans la démarche qualité des industries pharmaceutiques ou cosmétiques.

Par ailleurs, ces contrôles doivent répondre à un niveau d'exigence toujours plus important, de par une réglementation toujours plus stricte.

Parmi les outils à disposition des professionnels de santé ou des industriels pour la réalisation de contrôles environnementaux, les aérobiocollecteurs sont des solutions de choix pour la détection des microorganismes de l'air. Ces dispositifs sont placés aux endroits appropriés dans les lieux où l'on souhaite mesurer l'aérobio-contamination. Ils sont généralement constitués d'un collecteur d'air couplé à un milieu de culture. L'air collecté par le collecteur d'air entre en contact avec le milieu de culture; milieu de culture sur lequel viennent se déposer les microorganismes éventuellement contenus dans l'air collecté. Le milieu de culture est ensuite récupéré et placé à l'étuve pour favoriser la croissance des microorganismes. Il est ainsi possible de détecter et d'identifier lesdits microorganismes par des techniques traditionnelles de microbiologie.

Ces dispositifs présentent néanmoins un inconvénient majeur qui est lié à la technologie utilisée. Cet inconvénient est le temps nécessaire pour obtenir le résultat d'analyse. En effet, l'utilisation des techniques traditionnelles de microbiologie, en particulier de bactériologie, implique le respect de temps d'incubation nécessaires à la croissance cellulaire, voire des phases de re-ensemencement sur des milieux de culture spécifiques pour permettre l'identification. Il s'ensuit que le temps nécessaire pour obtenir un résultat est relativement long, voire trop long, quand on cherche à détecter et identifier un organisme pathogène, responsable d'une infection nosocomiale ou d'une toxi-infection alimentaire.

Un autre inconvénient de ce type de dispositif est que l'utilisation de milieux de culture, s'il permet de discriminer entre les genres et espèces bactériennes, ne permet généralement pas de discriminer les souches d'une même espèce bactérienne. Or, l'on sait que la pathogénicité d'un microorganisme peut varier de manière significative selon la souche considérée.

Il existe par ailleurs des dispositifs destinés à la récupération des particules présentes dans l'air, en particulier des microorganismes. Le document GB-2 254 024 décrit ainsi un dispositif pour collecter les particules contenues dans l'air dont le principe est basé sur l'effet cyclone. Si un tel dispositif se montre approprié à la collecte des particules contenues dans l'air, y compris les microorganismes, il n'est en aucun cas étudié pour traiter l'échantillon ainsi obtenu, en particulier pour réaliser l'extraction du matériel génétique destiné à être utilisé pour l'analyse.

De manière plus générale, les techniques les plus pertinentes en termes d'identification de microorganismes et/ou de rapidité de rendu des résultats, que ce soit vis-à-vis d'échantillons cliniques ou environnementaux, sont sans nul doute les techniques de diagnostic moléculaire. Ces techniques basées sur l'analyse du matériel génétique des microorganismes, et en particulier de certaines séquences spécifiques d'intérêt, permettent d'obtenir une identification très pointue des microorganismes en un temps record, puisqu'elles permettent de s'affranchir des étapes de culture.

Néanmoins, l'utilisation de telles techniques présente un certain nombre de limites, parmi lesquelles la plus importante est la quantité potentiellement limitée de microorganismes présents dans l'air et donc récupérable pour réaliser l'analyse. En effet, on sait que les prélèvements environnementaux, mais aussi certains prélèvements cliniques sont relativement pauvres en microorganismes. Il s'ensuit que la quantité de matériel génétique obtenue à partir de cette matière première est faible. Les performances de la technique utilisée pour extraire les acides nucléiques, en termes de rendement, deviennent alors un paramètre crucial.

Par ailleurs, la plupart des techniques existantes de lyse de microorganismes, sont longues, nécessitent l'intervention de personnel qualifié pour prendre en charge les étapes manuelles.

Le document WO-A-2005/038025 décrit une méthode d'extraction d'acides nucléiques de microorganismes prélevés notamment dans l'air. Cette méthode consiste dans la mise en œuvre de trois méthodes de lyse différentes, à savoir une lyse chimique, une lyse par choc thermique et une lyse mécanique. Si une telle méthode permet sans nul doute d'optimiser le rendement d'extraction des acides nucléiques et donc d'augmenter la quantité de matériel génétique disponible pour l'analyse, il n'en demeure pas moins que ce rendement reste dépendant de la quantité de microorganismes récupérés. Or, rien dans ce document n'est décrit pour optimiser la récupération desdits microorganismes.

Le document US-5,707,861 décrit un dispositif permettant de désintégrer des cellules vivantes du type microorganismes. Ce dispositif permet de lyser les cellules en utilisant à la fois des billes de verre mais également l'effet de vibration dû à l'espace existant entre les tubes contenant les microorganismes et les trous du support portant lesdits tubes. Ainsi, un tel dispositif permet d'optimiser la lyse de cellules et donc d'optimiser l'extraction du matériel génétique. Un tel dispositif et la méthode mise en œuvre par ce dernier présentent les mêmes limites que celles exposées précédemment, à savoir qu'ils restent dépendants de la quantité de microorganismes récupérés. Par ailleurs, ils présentent comme inconvénient supplémentaire de devoir effectuer subséquemment une étape de concentration des acides nucléiques afin de les isoler des débris cellulaires. Ils nécessitent enfin de récupérer manuellement les acides nucléiques, à l'issue de l'étape de concentration.

Ces problèmes se posent également avec le dispositif décrit dans le document US-5,567,050.

Des systèmes plus intégrés ont également été décrits. Ainsi, le document WO-A-2004/018704 décrit un dispositif et une méthode utilisant la technique d'amplification PCR (Polymerase Chain Reaction) pour collecter des microorganismes dans l'air et les identifier. Ce système est spécialement adapté à la lutte contre les tentatives d'attentats par contamination biologique dans les centres de tri postal. Ce système se compose d'un dispositif de collecte d'air placé le long du circuit de transport du courrier, un dispositif de filtration/séparation des particules par effet cyclone, un dispositif de concentration/récupération des particules dans un échantillon liquide, un dispositif de transfert d'une fraction de l'échantillon dans une cartouche d'analyse par PCR GeneXpert™ de la société Cepheid. La cartouche est ensuite transférée manuellement dans un automate d'analyse biologique indépendant à fin d'identification du ou des microorganismes collectés dans l'air.

Si ce système permet de résoudre un bon nombre de problèmes techniques liés aux dispositifs et procédés décrits précédemment, il présente nonobstant des inconvénients majeurs. Le premier de ces inconvénients est que le système de traitement de l'échantillon (collecte, séparation, concentration/récupération) préalablement au transfert dans la cartouche d'analyse est relativement complexe et encombrant. Un deuxième inconvénient est que les microorganismes collectés sont récupérés dans un échantillon liquide dont seule une fraction est analysée. Ce qui signifie que le risque de ne pas récupérer la totalité des microorganismes et donc la totalité des acides nucléiques est très important, limitant fortement la pertinence de l'analyse. Par ailleurs, malgré sa complexité, ce système nécessite le transfert manuel de la cartouche dans l'automate d'analyse GeneXpert™.

Ainsi, un premier objectif de la présente invention est de fournir une cartouche, un dispositif et des méthodes selon les revendications 1-31, à la fois efficaces pour des prélèvements environnementaux et cliniques, pour une grande diversité de microorganismes, qu'il s'agisse de bactéries, de virus ou encore de champignons, éventuellement à l'état végétatif ou sous forme de spores.

Un autre objectif de la présente invention est de fournir un dispositif apte à lyser efficacement lesdits microorganismes contenus dans un prélèvement environnemental tel que l'air, ou dans un prélèvement clinique, afin d'en extraire les acides nucléiques et récupérer lesdits acides nucléiques à fin d'analyse, de manière intégrée.

Un autre objectif de la présente invention est de fournir un dispositif apte à collecter l'intégralité des microorganismes contenus dans un prélèvement d'air.

Un autre objectif de la présente invention est de fournir un dispositif simple de conception.

Un autre objectif de la présente invention est de fournir un dispositif excessivement compact.

Un autre objectif de la présente invention est de fournir un dispositif clos dans lequel les différentes étapes énoncées ci-dessus se déroulent sans risque de contamination extérieure.

Un autre objectif de la présente invention est de fournir un dispositif dans lequel lesdites étapes se déroulent sans transfert de l'échantillon par l'opérateur, empêchant par là même la contamination de ce dernier.

Enfin, un autre objectif de la présente invention est de fournir un dispositif en mesure de fournir des acides nucléiques cibles dans un tampon apte à être directement utilisé dans des étapes de diagnostic moléculaire comprenant par exemple des étapes d'amplification et de détection et ce, sans étapes de prétraitement additionnelles telles qu'une centrifugation ou une filtration.

Ces objectifs parmi d'autres sont atteints par la présente invention qui concerne en premier lieu, une cartouche, selon la revendication 1.

Selon une variante de la cartouche des revendications 1 et 2, celle-ci comporte un moyen de connexion à un dispositif d'analyse.

De façon préférentielle, le diamètre des billes est compris entre 200 et 600 µm.

L'invention concerne également, un dispositif de collecte de microorganismes contenus dans l'air, ledit dispositif comportant :
- un moyen de collecte d'air, comprenant un élément supérieur comportant un conduit d'admission de l'air et un élément inférieur comportant un conduit d'évacuation de l'air, lesdits éléments supérieur et inférieur pouvant être solidarisés l'un à l'autre de sorte qu'un courant d'air puisse être créé à l'intérieur dudit moyen de collecte d'air ;
- une cartouche, selon les revendications 1-4, ladite cartouche étant positionnée à l'intérieur dudit moyen de collecte d'air.

Le moyen de collecte d'air est apte à être connecté à un circuit de recyclage de l'air.

L'invention concerne en outre un dispositif de lyse de microorganismes, dans le but d'isoler les acides nucléiques desdits microorganismes, ledit dispositif comportant :
- une cartouche selon les revendications 1-4, ladite cartouche comportant des microorganismes disposés dans la zone de rétention des microorganismes ;
- un moyen de récupération des acides nucléiques, de forme sensiblement cylindrique, apte à être emboîté dans la cartouche, ledit moyen de récupération, coopérant avec les moyens de lyse des microorganismes afin de lyser lesdits microorganismes et permettre la libération des acides nucléiques.

Avantageusement, le moyen de récupération des acides nucléiques comporte un moyen d'aspiration/refoulement de liquide.

De façon remarquable, le moyen de récupération des acides nucléiques comporte, en outre, une zone de stockage d'un liquide.

Selon un mode préférentiel de réalisation, le diamètre interne de la cartouche est supérieur au diamètre externe du moyen de récupération des acides nucléiques, de sorte que lorsque le moyen de récupération des acides nucléiques est emboîté dans la cartouche, la distance séparant la paroi interne de la cartouche de la paroi externe du moyen de récupération des acides nucléiques soit suffisamment importante pour permettre aux moyens de lyse de venir se positionner dans cet espace interstitiel et suffisamment faible pour que les moyens de lyse soient au contact de l'une ou l'autre desdites parois.

L'invention concerne également un procédé de concentration des microorganismes contenus dans l'air, ledit procédé comprenant les étapes consistant à :
a) Placer une cartouche selon les revendications 1-4 à l'intérieur du moyen de collecte d'air, de sorte que la zone de rétention, à l'intérieur de ladite cartouche, soit en communication avec le conduit d'admission d'air du moyen de collecte d'air,
b) Provoquer l'entrée d'air à l'intérieur dudit moyen de collecte d'air par tout moyen approprié,
c) Concentrer les microorganismes contenus dans l'air dans la zone de rétention de la cartouche.

Selon un mode de réalisation particulier, il comporte en outre une étape d) consistant à faire croître les microorganismes dans la zone de rétention.

Avantageusement, les microorganismes sont retenus sur les moyens de lyse, présents dans la zone de rétention.

L'invention concerne en outre un procédé de lyse de microorganismes contenus dans l'air, ledit procédé comportant les étapes consistant à :
a) Placer une cartouche selon les revendications 1-4 à l'intérieur du moyen de collecte d'air, de sorte que la zone de rétention, à l'intérieur de ladite cartouche, soit en communication avec le conduit d'admission d'air du moyen de collecte d'air,
b) Provoquer l'entrée d'air à l'intérieur dudit moyen de collecte d'air par tout moyen approprié,
c) Concentrer les microorganismes contenus dans l'air, dans la zone de rétention de la cartouche,
d) Sortir la cartouche du moyen de collecte d'air,
e) Placer le moyen de récupération des acides nucléiques dans la cartouche,
f) Introduire un liquide d'intérêt dans la cartouche, entraînant la mise en suspension des moyens de lyse situés dans la zone de rétention des microorganismes de la cartouche, et
g) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes.

L'invention concerne encore un procédé de lyse de microorganismes contenus dans l'air, ledit procédé comportant les étapes consistant à :
a) Placer une cartouche selon les revendications 1-4 à l'intérieur du moyen de collecte d'air, de sorte que la zone de rétention, à l'intérieur de ladite cartouche, soit en communication avec le conduit d'admission d'air du moyen de collecte d'air,
b) Provoquer l'entrée d'air à l'intérieur dudit moyen de collecte d'air par tout moyen approprié,
c) Concentrer les microorganismes contenus dans l'air, dans la zone de rétention de la cartouche,
d) Sortir la cartouche du moyen de collecte d'air,
e) Placer le moyen de récupération des acides nucléiques dans la cartouche par emboîtement,
f) Provoquer le refoulement d'un liquide d'intérêt placé préalablement dans la zone de stockage du moyen de récupération des acides nucléiques, ledit refoulement étant obtenu par le biais du moyen d'aspiration/refoulement du moyen de récupération des acides nucléiques, le liquide ainsi refoulé venant remplir l'espace interstitiel situé entre le moyen de récupération des acides nucléiques et la cartouche, entraînant la mise en suspension des moyens de lyse situés dans la zone de rétention des microorganismes de la cartouche, lesdits moyens de lyse venant se positionner entre la paroi intérieure verticale de la cartouche et la paroi extérieure verticale du moyen de récupération des acides nucléiques, et
g) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes.

Un autre objet de l'invention concerne un procédé d'extraction des acides nucléiques de microorganismes contenus dans l'air, ledit procédé comportant les étapes consistant à :
a) Placer une cartouche selon les revendications 1-4 à l'intérieur du moyen de collecte d'air, de sorte que la zone de rétention, à l'intérieur de ladite cartouche, soit en communication avec le conduit d'admission d'air du moyen de collecte d'air,
b) Provoquer l'entrée d'air à l'intérieur dudit moyen de collecte d'air par tout moyen approprié,
c) Concentrer les microorganismes contenus dans l'air, dans la zone de rétention de la cartouche,
d) Sortir la cartouche du moyen de collecte d'air,
e) Placer le moyen de récupération des acides nucléiques dans la cartouche,
f) Introduire un liquide d'intérêt dans la cartouche, entraînant la mise en suspension des moyens de lyse situés dans la zone de rétention des microorganismes de la cartouche, et
g) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes, et
h) Aspirer le liquide d'intérêt contenant les acides nucléiques desdits microorganismes libérés lors de la lyse.

L'invention concerne également un procédé d'extraction des acides nucléiques de microorganismes contenus dans l'air, ledit procédé comportant les étapes consistant à :
a) Placer une cartouche selon les revendications 1-4 à l'intérieur du moyen de collecte d'air, de sorte que la zone de rétention, à l'intérieur de ladite cartouche, soit en communication avec le conduit d'admission d'air du moyen de collecte d'air,
b) Provoquer l'entrée d'air à l'intérieur dudit moyen de collecte d'air par tout moyen approprié,
c) Concentrer les microorganismes contenus dans l'air, dans la zone de rétention de la cartouche,
d) Sortir la cartouche du moyen de collecte d'air,
e) Placer le moyen de récupération des acides nucléiques dans la cartouche par emboîtement,
f) Provoquer le refoulement d'un liquide d'intérêt placé préalablement dans la zone de stockage du moyen de récupération des acides nucléiques, ledit refoulement étant obtenu par le biais du moyen d'aspiration/refoulement du moyen de récupération des acides nucléiques, le liquide ainsi refoulé venant remplir l'espace interstitiel situé entre le moyen de récupération des acides nucléiques et la cartouche, entraînant la mise en suspension des moyens de lyse situés dans la zone de rétention des microorganismes de la cartouche, lesdits moyens de lyse venant se positionner entre la paroi intérieure verticale de la cartouche et la paroi extérieure verticale du moyen de récupération des acides nucléiques,
g) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes, libérant ainsi les acides nucléiques desdits microorganismes, et
h) Provoquer l'aspiration du liquide d'intérêt dans la zone de stockage du moyen de récupération des acides nucléiques, ladite aspiration étant obtenue par le biais du moyen d'aspiration/refoulement du moyen de récupération des acides nucléiques, le liquide d'intérêt ainsi aspiré contenant les acides nucléiques desdits microorganismes libérés lors de la lyse.

Ces procédés d'extraction comportent préférentiellement une étape supplémentaire d') consistant à faire croître les microorganismes concentrés dans la zone de rétention de la cartouche.

Cette mise en croissance est obtenue par incubation de la cartouche dans une étuve pendant un temps allant de 2 à 24 heures.

Un autre objet de l'invention concerne un procédé de lyse de microorganismes contenus dans l'air, ledit procédé comportant les étapes consistant à :
a) Obtenir une cartouche selon les revendications 1-4, dans laquelle des microorganismes sont disposés au voisinage de la zone de rétention,
b) Placer le moyen de récupération des acides nucléiques dans la cartouche par emboîtement,
c) Provoquer le refoulement d'un liquide d'intérêt placé préalablement dans la zone de stockage du moyen de récupération des acides nucléiques, ledit refoulement étant obtenu par le biais du moyen d'aspiration/refoulement du moyen de récupération des acides nucléiques, le liquide ainsi refoulé venant remplir l'espace interstitiel situé entre le moyen de récupération des acides nucléiques et la cartouche, entraînant la mise en suspension des moyens de lyse situés dans la zone de rétention des microorganismes de la cartouche, lesdits moyens de lyse venant se positionner entre la paroi intérieure verticale de la cartouche et la paroi extérieure verticale du moyen de récupération des acides nucléiques, et
d) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes.

Un autre objet de l'invention concerne un procédé de lyse de microorganismes, ledit procédé comportant les étapes consistant à :
a) Obtenir une cartouche selon les revendications 1-4, dans laquelle des microorganismes sont concentrés dans la zone de rétention,
b) Placer le moyen de récupération des acides nucléiques dans la cartouche,
c) Introduire un liquide d'intérêt dans la cartouche, entraînant la mise en suspension des moyens de lyse situés dans la zone de rétention des microorganismes de la cartouche, et
d) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes.

Un autre objet de l'invention concerne un procédé d'extraction des acides nucléiques de microorganismes, ledit procédé comportant les étapes consistant à :
a) Obtenir une cartouche selon les revendications 1-4, dans laquelle des microorganismes sont concentrés dans la zone de rétention,
b) Placer le moyen de récupération des acides nucléiques dans la cartouche par emboîtement,
c) Provoquer le refoulement d'un liquide d'intérêt placé préalablement dans la zone de stockage du moyen de récupération des acides nucléiques, ledit refoulement étant obtenu par le biais du moyen d'aspiration/refoulement du moyen de récupération des acides nucléiques, le liquide ainsi refoulé venant remplir l'espace interstitiel situé entre le moyen de récupération des acides nucléiques et la cartouche, entraînant la mise en suspension des moyens de lyse situés dans la zone de rétention des microorganismes de la cartouche, lesdits moyens de lyse venant se positionner entre la paroi intérieure verticale de la cartouche et la paroi extérieure verticale du moyen de récupération des acides nucléiques,
d) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes, libérant ainsi les acides nucléiques desdits microorganismes, et
e) Provoquer l'aspiration du liquide d'intérêt dans la zone de stockage du moyen de récupération des acides nucléiques, ladite aspiration étant obtenue par le biais du moyen d'aspiration/refoulement du moyen de récupération des acides nucléiques, le liquide d'intérêt ainsi aspiré contenant les acides nucléiques desdits microorganismes libérés lors de la lyse.

Un autre objet de l'invention concerne un procédé d'extraction des acides nucléiques de microorganismes, ledit procédé comportant les étapes consistant à :
a) Obtenir une cartouche selon les revendications 1-4, dans laquelle des microorganismes sont concentrés dans la zone de rétention,
b) Placer le moyen de récupération des acides nucléiques dans la cartouche,
c) Introduire un liquide d'intérêt dans la cartouche, entraînant la mise en suspension des moyens de lyse situés dans la zone de rétention des microorganismes de la cartouche, et
d) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes, et
e) Aspirer le liquide d'intérêt contenant les acides nucléiques desdits microorganismes libérés lors de la lyse.

L'invention concerne également un procédé de lyse de microorganismes, ledit procédé comportant les étapes consistant à :
a) Introduire un échantillon liquide contenant lesdits microorganismes dans une cartouche selon les revendications 1-4, au voisinage de la zone de rétention, de sorte que ledit échantillon liquide entraîne la mise en suspension des moyens de lyse situés dans ladite zone de rétention des microorganismes de la cartouche,
b) Placer le moyen de récupération des acides nucléiques dans la cartouche,
c) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes.

Par échantillon liquide, on entend tout échantillon liquide susceptible de contenir des microorganismes. Il peut s'agir d'un échantillon liquide d'origine humaine ou animal. Cet échantillon peut être par exemple, de l'urine, du sang total, du plasma ou tout autre liquide corporel. L'échantillon liquide peut être d'origine alimentaire tel qu'une boisson. Il peut être également d'origine environnementale, tel que de l'eau. Par ailleurs, l'échantillon liquide peut être également un liquide dit de transfert, dans lequel des éventuels microorganismes contenus sur un dispositif de prélèvement de surface, du type écouvillon tel que ceux commercialisés par la société COPAN, sous la dénomination flockedSWABS, ont été resuspendus par agitation dudit écouvillon dans ledit liquide de transfert.

En outre, l'invention concerne un procédé d'extraction des acides nucléiques de microorganismes, ledit procédé comportant les étapes consistant à :
a) Introduire un échantillon liquide contenant lesdits microorganismes dans une cartouche selon les revendications 1-4, au voisinage de la zone de rétention, de sorte que ledit échantillon liquide entraîne la mise en suspension des moyens de lyse situés dans ladite zone de rétention des microorganismes de la cartouche,
b) Placer le moyen de récupération des acides nucléiques dans la cartouche,
c) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes,
d) Aspirer le liquide d'intérêt contenant les acides nucléiques desdits microorganismes libérés lors de la lyse.

Un autre objet de l'invention concerne en outre un procédé d'identification d'un ou plusieurs microorganismes comprenant les étapes consistant à :
a) Isoler les acides nucléiques des microorganismes contenus dans ledit prélèvement au moyen selon les revendications 5-10,
b) Identifier le ou les microorganismes ainsi isolés.

Selon une variante avantageuse du procédé d'identification selon l'invention, celui-ci comporte en outre une étape intermédiaire consistant à purifier les acides nucléiques. Cette étape de purification, permettant la séparation entre les acides nucléiques et les autres constituants cellulaires relargués dans l'étape de lyse. Cette étape permet généralement de concentrer les acides nucléiques, et peut être adaptée à la purification d'ADN ou d'ARN. A titre d'exemple, on peut utiliser des particules magnétiques éventuellement revêtues d'oligonucléotides, par adsorption ou covalence (voir à ce sujet les brevets US 4,672,040 et US 5,750,338), et ainsi purifier les acides nucléiques qui se sont fixés sur ces particules magnétiques, par une étape de lavage. Cette étape de purification des acides nucléiques est particulièrement intéressante si l'on souhaite amplifier ultérieurement lesdits acides nucléiques. Un mode de réalisation particulièrement intéressant de ces particules magnétiques est décrit dans les demandes de brevet: WO-A-97/45202 et WO-A-99/35500. Un autre exemple intéressant de méthode de purification des acides nucléiques est l'utilisation de silice soit sous forme de colonne, soit sous forme de particules inertes (Boom R. et al., J. Clin. Microbiol., 1990, n°28(3), p. 495-503) ou magnétiques (Merck: MagPrep^{→} Silica, Promega: MagneSil™ Paramagnetic particles). D'autres méthodes très répandues reposent sur des résines échangeuses d'ions en colonne ou en format particulaire paramagnétique (Whatman: DEAE-Magarose) (Levison PR et al., J. Chromatography, 1998, p. 337-344). Une autre méthode très pertinente mais non exclusive pour l'invention est celle de l'adsorption sur support d'oxyde métallique (société Xtrana: matrice Xtra-Bind™).

De façon particulière, l'étape d'identification comporte les sous-étapes consistant à :
a) amplifier de façon spécifique les acides nucléiques isolés,
b) détecter les acides nucléiques ainsi amplifiés.

Selon une variante préférentielle du procédé d'identification, l'étape identification est réalisée dans un dispositif d'identification, en communication fluidique avec la cartouche du dispositif selon l'invention.

Ainsi, les acides nucléiques isolés sont transférés du moyen de récupération des acides nucléiques du dispositif selon l'invention, vers le dispositif d'identification.

Le transfert des acides nucléiques est avantageusement obtenu par refoulement du liquide d'intérêt contenant les acides nucléiques, ledit liquide d'intérêt étant contenu dans la zone de stockage du moyen de récupération des acides nucléiques, par le biais du moyen d'aspiration/refoulement du moyen de récupération des acides nucléiques.

Les microorganismes sont pris dans le groupe comprenant les bactéries, les virus, les levures, les moisissures, les parasites.

Les échantillons à partir desquels sont isolés les microorganismes sont d'origine environnementale. Ainsi, il peut s'agir d'un échantillon d'air ou de liquide, tel que de l'eau ; des prélèvement de surface. Les échantillons peuvent être également d'origine clinique, à savoir tout échantillon d'origine humaine ou animale, apte à faire l'objet d'une analyse pour la rechercher et l'identification d'un microorganisme, éventuellement pathogène.

La présence des acides nucléiques cibles est mise en évidence par la visualisation de réactions d'hybridation. On entend par réaction d'hybridation toute réaction entre un acide nucléique de capture et un acide nucléique cible isolé ou généré par une étape de transcription, de transcription inverse ou d'amplification de type NASBA (pour Nucleic Acid Sequence Based Amplification en anglais) ou PCR (pour Polymerase Chain Reaction en anglais).

On entend par acide nucléique, les oligonucléotides, les acides désoxyribonucléiques et les acides ribonucléiques, ainsi que leurs dérivés. Le terme oligonucléotide désigne un enchaînement d'au moins deux nucléotides (désoxyribonucléotides ou ribonucléotides, ou les deux), naturels ou modifiés, susceptibles de s'hybrider, dans des conditions appropriées d'hybridation, avec un oligonucléotide au moins partiellement complémentaire. Par nucléotide modifié, on entend par exemple un nucléotide comportant une base modifiée et/ou comportant une modification au niveau de la liaison internucléotidique et/ou au niveau du squelette. A titre d'exemple de base modifiée, on peut citer l'inosine, la méthyl-5-désoxycytidine, la diméthylamino-5-désoxyuridine, la diamino-2,6-purine et la bromo-5-désoxyuridine.

Pour illustrer une liaison internucléotidique modifiée, on peut mentionner les liaisons phosphorothioate, N-alkylphosphoramidate, alkylphosphonate et alkylphosphodiester.

Les alpha-oligonucléotides tels que ceux décrits dans FR-A-2 607 507, les LNA tels que phosphorothioate-LNA and 2'-thio-LNA décrits dans Bioorganic & Médicinal Chemistry Letters, Volume 8, Issue 16,18 August 1998 ,pages 2219-2222, et les PNA qui font l'objet de l'article de M. Egholm et al., J. Am. Chem. Soc. (1992), 114,1895- 1897, sont des exemples d'oligonucléotides constitués de nucléotides dont le squelette est modifié.

La visualisation des réactions d'hybridation peut être effectuée par tout moyen de détection, tels que des moyens directs ou indirects.

Dans le cas de la détection directe, c'est-à-dire sans l'intermédiaire d'un marquage, on observe les réactions d'hybridation par résonance plasmon ou par voltamétrie cyclique sur une électrode portant un polymère conducteur.

Dans le cas de la détection indirecte, c'est-à-dire par l'intermédiaire d'un marquage, le marquage peut être effectué soit directement sur les acides nucléiques cibles, soit par l'intermédiaire d'un partenaire de liaison spécifique desdits acides nucléiques préalablement marqué.

On entend par partenaire de liaison spécifique des acides nucléiques cibles tout partenaire capable de se lier avec l'acide nucléique cible et on donnera à titre d'exemples les acides nucléiques, les oligonucléotides ou polynucléotides et les substrats enzymatiques.

Par marquage, on entend la fixation d'un marqueur capable de générer directement ou indirectement un signal détectable. Une liste non limitative de ces marqueurs consiste en : les enzymes qui produisent un signal détectable par exemple par électrochimie, colorimétrie, fluorescence, luminescence, enzymes comme la péroxydase de Raifort (HRP), la phosphatase alcaline (PAL), la α-galactosidase, la glucose-6-phosphate déshydrogénase ; les inhibiteurs d'enzymes ; les co-facteurs d'enzymes ; les particules telles que les particules en or, les latex magnétiques, les liposomes ; les chromophores comme les composés, luminescents, colorants, les molécules radioactives comme le ³²P, le ³⁵S ou le ¹²⁵I, les molécules fluorescentes telles que la fluorescéine, la rhodamine, les Alexa®, l'umbelliférone, le luminol ou les phycocyanines. Dans le cas de la fluorescence, il peut s'agir du produit fluorescent d'une réaction enzyme-substrat, d'une combinaison fluorophore-quencher, d'une extinction de fluorescence ou de tout autre système basé sur des propriétés de fluorescence.

Des systèmes indirects peuvent être aussi utilisés, comme par exemple par l'intermédiaire d'un autre couple ligand/antiligand. Les couples ligand/antiligand sont bien connus de l'homme du métier, et on peut citer par exemple les couples suivants : biotine/streptavidine, sucre/lectine, polynucléotide/complémentaire du polynucléotide. Dans ce cas, c'est le ligand qui porte l'agent de liaison. L'antiligand peut être détectable directement par les marqueurs décrits au paragraphe précédent ou être lui-même détectable par un ligand/anti-ligand.

Ces systèmes de détection indirects peuvent conduire, dans certaines conditions, à une amplification du signal. Cette technique d'amplification du signal est bien connue de l'homme du métier, et l'on pourra se reporter aux demandes de brevet antérieures FR-A-2 781 802 ou WO-A-95/08000 de la demanderesse ou à l'article J. Histochem. Cytochem. 45 : 481-491, 1997.

Le marquage préalable des acides nucléiques cibles peut être effectué par incorporation directe ou indirecte de marqueur par une polymérase, par une kinase, de façon aléatoire ou spécifique, aux extrémités ou par incorporation « à l'intérieur » de la molécule.

Le marquage des partenaires de liaison spécifiques des analytes cibles est largement connu de l'homme du métier et est décrit par exemple par Greg T. Hermanson dans Bioconjugate Techniques, 1996, Academic Press Inc, 525B Street, San Diego, CA92101 USA.

Selon le type de marquage du conjugué utilisé, comme par exemple en utilisant une enzyme, l'homme du métier ajoutera des réactifs permettant la visualisation du marquage. Cette étape correspond à la révélation. Elle est précédée de l'utilisation d'un tampon de lavage qui permet d'éliminer les fractions d'analytes ou d'éléments non engagés dans la réaction, ou faiblement ou non spécifiquement liés, afin de limiter le bruit de fond.

Les buts et avantages du dispositif selon la présente invention seront mieux compris à la lumière de l'exemple nullement limitatif qui suit, en référence au dessin, dans lequel :
La figure 1 représente une vue éclatée en coupe longitudinale du moyen de collecte d'air, selon un premier mode de réalisation de l'invention.
La figure 2 représente une vue en coupe longitudinale du moyen de collecte d'air dans lequel a été placée une cartouche, pendant l'étape de collecte des microorganismes, selon un premier mode de réalisation de l'invention.
La figure 3 représente une vue en coupe longitudinale de la cartouche et du moyen de récupération des acides nucléiques à un stade initial du processus d'emboîtement dudit moyen à l'intérieur de la cartouche, selon un premier mode de réalisation de l'invention.
La figure 4 représente une vue en coupe longitudinale de la cartouche et du moyen de récupération des acides nucléiques à un stade avancé du processus d'emboîtement dudit moyen à l'intérieur de la cartouche, selon un premier mode de réalisation de l'invention.
La figure 5 représente une vue en coupe longitudinale de la cartouche et du moyen de récupération des acides nucléiques au stade final du processus d'emboîtement dudit moyen à l'intérieur de la cartouche, selon un premier mode de réalisation de l'invention.
La figure 6 représente une vue en coupe longitudinale du moyen de récupération des acides nucléiques emboîté dans la cartouche, pendant l'étape de lyse mécanique des microorganismes, selon un premier mode de réalisation de l'invention.
La figure 7 représente une vue en coupe longitudinale du moyen de récupération des acides nucléiques emboîté dans la cartouche, pendant l'étape d'aspiration du liquide d'intérêt contenant les acides nucléiques, dans la zone de stockage du moyen de récupération des acides nucléiques, selon un premier mode de réalisation de l'invention.
La figure 8 représente une vue en coupe longitudinale du moyen de récupération des acides nucléiques emboîté dans la cartouche, lorsque tout le liquide d'intérêt contenant les acides nucléiques a été aspiré dans la zone de stockage du moyen de récupération des acides nucléiques, selon un premier mode de réalisation de l'invention.
La figure 9 représente une vue en coupe longitudinale du moyen de récupération des acides nucléiques emboîté dans la cartouche, lors de la présentation du moyen d'identification des microorganismes, selon un premier mode de réalisation de l'invention.
La figure 10 représente une vue en coupe longitudinale du moyen de récupération des acides nucléiques emboîté dans la cartouche, une fois le moyen d'identification des microorganismes en communication fluidique avec la cartouche, selon un premier mode de réalisation de l'invention.
La figure 11 représente une vue en coupe longitudinale de l'ensemble moyen de récupération des acides nucléiques - cartouche - moyen d'identification des microorganismes, dans la phase initiale de transfert du liquide d'intérêt contenant les acides nucléiques, dans le moyen d'identification des microorganismes, selon un premier mode de réalisation de l'invention.
La figure 12 représente une vue en coupe longitudinale de l'ensemble moyen de récupération des acides nucléiques - cartouche - moyen d'identification des microorganismes, après transfert du liquide d'intérêt contenant les acides nucléiques, dans le moyen d'identification des microorganismes, selon un premier mode de réalisation de l'invention.
La figure 13 représente une vue en coupe longitudinale du moyen de collecte d'air dans lequel a été placée une cartouche, pendant l'étape de collecte des microorganismes, selon un deuxième mode de réalisation.
La figure 14 représente une vue en coupe longitudinale de la cartouche, pendant l'étape de distribution manuelle du liquide d'intérêt, selon le deuxième mode de réalisation.
La figure 15 représente une vue en coupe longitudinale du moyen de récupération des acides nucléiques placé dans la cartouche, pendant l'étape de lyse mécanique des microorganismes, selon le deuxième mode de réalisation.
La figure 16 représente une vue en coupe longitudinale du moyen de récupération des acides nucléiques placé dans la cartouche, pendant l'étape d'aspiration du liquide d'intérêt contenant les acides nucléiques, selon le deuxième mode de réalisation.
La figure 17 représente un schéma fonctionnel d'un banc d'essai destiné à tester la capacité de capture de bactéries contenues dans des aérosols, avec le dispositif selon l'invention.
La figure 18 est un graphique montrant l'efficacité de collecte du dispositif selon l'invention en fonction de la taille des particules d'aérosols produites.
La figure 19 est un graphique comparant l'efficacité de lyse de bactéries avec le dispositif selon l'invention et des méthodes alternatives de l'état de la technique.
La figure 20 est un graphique montrant la détection en temps réel par la technologie Real Time NASBA, des bactéries contenues dans différentes suspensions, après lyse de ces bactéries et récupération des acides nucléiques à l'aide du dispositif selon l'invention.
La figure 21 est un graphique montrant la détection en temps réel par la technologie Real Time NASBA, des bactéries contenues dans différents échantillons de sang total, après lyse de ces bactéries et récupération des acides nucléiques à l'aide du dispositif selon l'invention.

Selon un premier mode de réalisation, le premier élément constituant le dispositif selon l'invention est un moyen de collecte d'air 10. Ce moyen est composé d'un élément supérieur 12 et d'un élément inférieur 14. L'élément supérieur 12 est de forme générale cylindrique. L'extrémité inférieure de ce cylindre est libre, alors que l'extrémité supérieure est partiellement obturée, au moyen d'une paroi horizontale 13. Cette paroi 13 présente en son centre un orifice se prolongeant à l'intérieur de l'élément supérieur 12, par un conduit 18 dont la base est de forme sensiblement conique. Cette partie constitue en fait, le conduit d'admission de l'air à l'intérieur du moyen de collecte d'air 10. Selon un mode de réalisation particulier, ce conduit d'admission d'air peut être connecté à une canalisation d'un circuit de recyclage d'air par tout moyen approprié.

Le moyen de collecte d'air 10 peut par exemple avoir un diamètre extérieur compris entre 10 et 40 mm, préférentiellement 20 mm. Le diamètre interne du conduit d'admission de l'air est par exemple de 6 mm.

Ce moyen de collecte d'air 10 peut avantageusement être réalisé dans un matériau apte à être stérilisé, en particulier par autoclavage. Il peut ainsi être en métal, tel que l'aluminium ou l'acier. Il peut également être en polymère tel que le polyméthylméthacrylate (PMMA).

L'extrémité inférieure de la paroi circulaire verticale 20 de l'élément supérieur 12 comporte, sur sa face extérieure, un épaulement 201.Ce décrochement est réalisé sur tout le pourtour de la paroi 20. Il est destiné à faciliter la solidarisation de l'élément supérieur 12 et de l'élément inférieur 14.

L'élément inférieur 14 est également de forme générale cylindrique. L'extrémité supérieur de ce cylindre est libre, alors que l'extrémité inférieure est partiellement obturée, par une paroi horizontale 21 comportant en son centre un conduit d'évacuation de l'air 22, sensiblement cylindrique, la base dudit conduit étant solidaire de la paroi horizontale. L'extrémité inférieure du conduit 22 est libre. Ce conduit est en communication avec l'intérieur de l'élément inférieur 14, de sorte que, quand les éléments supérieur 12 et inférieur 14 du moyen de collecte d'air 10 sont solidarisés, le conduit 22 joue le rôle de conduit d'évacuation de l'air qui a été admis à l'intérieur du moyen de collecte d'air 10, par le biais du conduit d'admission de l'air 18.

L'extrémité supérieure de la paroi circulaire verticale 24 de l'élément inférieur 14 comporte, sur sa face intérieure, un épaulement 241. Cet épaulement est réalisé sur tout le pourtour de la paroi 24. Il est également destiné à faciliter la solidarisation de l'élément supérieur 12 et de l'élément inférieur 14, du fait que les extrémités des parois 20 et 24 présentent une section de forme complémentaire, facilitant l'emboîtement. Il importe en premier lieu que cette solidarisation soit réversible. Une fois emboîtés, les éléments 12 et 14 doivent pouvoir se déboîter.

Un moyen de solidarisation alternatif des éléments 12 et 14 peut être une solidarisation par vissage d'un élément sur l'autre. A cette fin, l'extrémité de la paroi d'un des éléments 12 ou 14 peut porter un filetage mâle et l'extrémité de la paroi du deuxième élément un filetage femelle correspondant.

L'important est que le moyen de collecte soit clos de façon hermétique, afin d'éviter toute entrée parasite d'air.

Selon un mode particulier d'utilisation du moyen de collecte de l'air, l'extrémité inférieure du conduit d'évacuation de l'air 22 peut être connectée à une pompe d'aspiration de l'air (non représentée) ou tout moyen de pompage équivalent. Cette pompe permet d'aspirer l'air ambiant à l'intérieur du moyen de collecte de l'air, lorsqu'on souhaite par exemple réaliser une analyse de l'air ambiant dans un environnement déterminé, tel qu'une chambre d'hôpital, une pièce de production de produits pharmaceutiques ou de produits agroalimentaires. A cette fin, il peut être préférable de disposer d'un moyen de pompage fonctionnant de manière autonome.

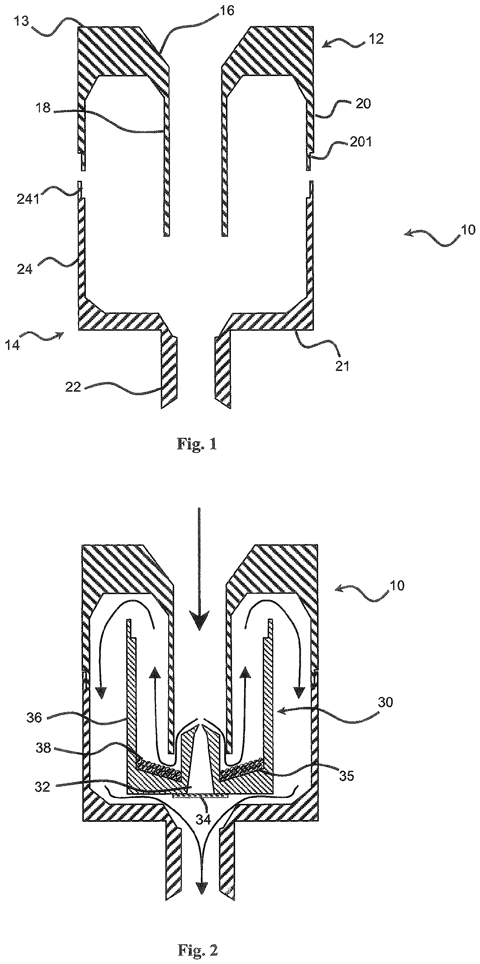

La figure 2 représente le moyen de collecte d'air 10, lors de son fonctionnement, combiné à une cartouche 30. Comme on peut le voir sur cette figure, la cartouche 30 est disposée à l'intérieur du moyen de collecte d'air 10. Pour ce faire, les éléments 12 et 14 constituant le moyen de collecte d'air 10 sont séparés. La cartouche 30 est placée en appui dans l'élément inférieur 14 du moyen de collecte d'air 10. L'élément supérieur 12 est alors repositionné sur l'élément inférieur 14 et ces deux moyens sont solidarisés. L'ensemble constitué par le moyen de collecte d'air 10 et la cartouche 30 est alors, soit connecté à un circuit de recyclage, soit à un dispositif de pompage, afin d'entraîner la circulation de l'air à l'intérieur du moyen de collecte d'air 10, tel que décrit *supra.*

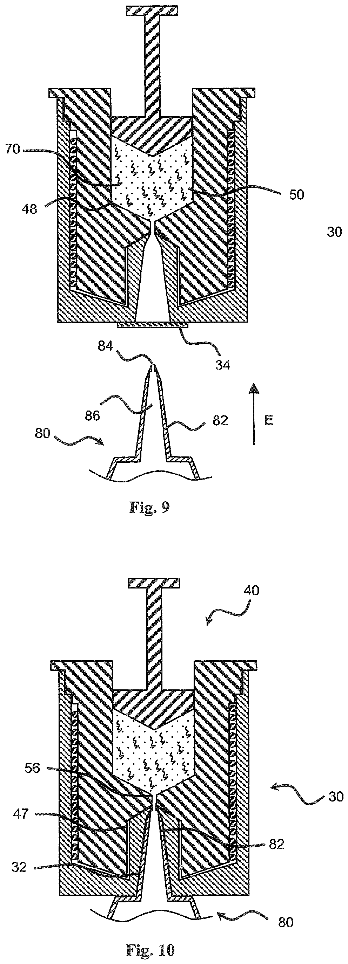

La cartouche 30 représentée sur la figure 2 en coupe longitudinale, a la forme générale d'un cylindre de section transversale sensiblement circulaire. L'extrémité supérieure du cylindre est libre, alors que l'extrémité inférieure est constituée par une paroi, présentant en son centre un orifice, dans le prolongement duquel se trouve un conduit 32 s'étendant à l'intérieur de la cartouche 30. On constate que la section intérieure du conduit tend à se réduire au fur et à mesure que l'on se rapproche de l'extrémité supérieure du conduit. On constate par ailleurs, que l'extrémité inférieure du conduit est obturée par une membrane 34, jouant le rôle de septum. Le matériau constituant la membrane 34 est adapté pour être déchiré par simple pression avec un objet pointu. Ce processus est décrit *infra* en relation avec les figures 9 et 10. Ce matériau est par exemple un polyéthylène téréphtalique (PET) ou un polycarbonate.

Comme on peut le voir sur la figure 2, la face interne de la paroi inférieure 35 de la cartouche 30 est inclinée, le point le plus bas de la pente se trouvant au contact du conduit 32 et le point le plus haut au contact de la paroi verticale 36 de la cartouche 30. Cette paroi 35 sert de support aux moyens de lyse 38 et constitue la zone de rétention des microorganismes. Ces moyens de lyse sont ici constitués par des billes de taille identique. Selon un mode de réalisation préférentielle, ces billes sont en verre. Elles pourraient néanmoins être constituées de tout autre matériau équivalen,t tel que le fer. Ces billes présentent un diamètre avantageusement compris entre 200 et 800 micromètres (µm).

Selon une variante avantageuse, les billes peuvent être de tailles différentes. Il peut être ainsi particulièrement approprié d'utiliser un mélange de billes de diamètre compris entre 200 et 300 µm avec des billes dont le diamètre est compris entre 400 et 600 µm.

Les billes sont maintenues en place sous la forme d'une ou plusieurs couches superposées au moyen d'un matériau gélifié, déposé sous forme de couche, dans laquelle la ou les couches de billes sont noyées. Le matériau gélifié doit répondre à plusieurs contraintes. La première est qu'il doit être inerte, afin de ne pas influer les procédés qui sont mis en oeuvre à l'intérieur de la cartouche. La deuxième est qu'il doit avoir la capacité de se dissoudre dans un liquide et ce, afin de libérer les particules qu'il emprisonne pour la mise en œuvre de la lyse des microorganismes. Un tel matériau peut par exemple être de l'agarose.

Alternativement, le matériau gélifié peut avantageusement être un milieu de culture pour les microorganismes. En effet, les milieux de culture gélosés sont classiquement utilisés dans le domaine du diagnostic in vitro et ce, depuis très longtemps. L'utilisation d'un tel milieu de culture présente plusieurs avantages. Le principal est de permettre une phase de croissance des microorganismes avant de réaliser la lyse de ces derniers. Même si le dispositif selon l'invention cherche à répondre à un besoin lié à la détection rapide de microorganismes pathogènes, il n'en demeure pas moins qu'une phase de croissance de quelques minutes à quelques heures permettrait une multiplication des microorganismes, ce qui a pour effet direct de disposer d'une plus grande quantité d'acides nucléiques. Un second avantage de l'utilisation de milieux de culture est que ces derniers peuvent être sélectifs pour une ou plusieurs espèces données. Il s'ensuit que l'utilisation de tels milieux peut permettre une croissance et donc une détection sélective de certains microorganismes pathogènes au détriment d'autres microorganismes sans intérêt, qui peuvent éventuellement interférer dans l'analyse. Ainsi, il peut être envisagé de disposer de plusieurs cartouches différenciées, chacune étant adaptée à la détection d'une espèce spécifique de microorganisme.

Les dimensions de la cartouche 30 peuvent par exemple être de 8 à 16 mm pour ce qui est du diamètre interne, préférentiellement 12 mm. L'épaisseur totale des couches de billes est typiquement comprise entre 1 et 2 mm, ce qui correspond à une quantité de billes de verre comprise entre 0,4 et 1 gramme.

La cartouche 30 est avantageusement réalisée en utilisant une technique de moulage par injection. Le matériau utilisé est par exemple du polypropylène, du polystyrène, du polycarbonate ou du PMMA

Lorsque la circulation d'air dans le moyen de collecte d'air est déclenchée, le chemin suivi par ce dernier est représenté par les flèches sur la figure 2. Ainsi, on constate que l'air entre dans le moyen de collecte d'air 10 par le conduit d'admission de l'air 18. Le conduit 32 de la cartouche 30 étant partiellement inséré à l'intérieur du conduit d'admission de l'air 18, le flux d'air dans ce dernier se transforme au sommet du conduit 32 en un flux périphérique. Ceci s'explique par le fait que l'extrémité supérieure du conduit 32 est de forme sensiblement conique. Par ailleurs, la présence de la membrane 34 à la base du conduit 32 empêche l'air de pénétrer dans le conduit 32, puisque très rapidement celui-ci se retrouve en surpression. L'espace interstitiel entre la paroi du conduit d'admission 18 et la paroi du conduit 32 se rétrécissant brusquement de par la forme conique de l'extrémité du conduit 32, il se produit une accélération du flux d'air périphérique. Il s'ensuit que ce dernier vient se briser sur la couche supérieure de billes 38, entraînant à cette endroit la rétention par impaction des microorganismes transportés dans le flux d'air, à la surface du matériau gélifié ou du milieu de culture. Un tel processus est donc ainsi relativement similaire à celui qui se déroule dans les aérobiocollecteurs.

L'air, quant à lui, poursuit sa route aspiré par le moyen de pompage. Il remonte le long de la face interne de la paroi verticale 36 de la cartouche 30, au niveau de laquelle il perd de la vitesse par cause d'élargissement du chemin de circulation. Il redescend le long de la face externe de cette même paroi et se transforme à nouveau en un flux centralisé à l'entrée du conduit d'évacuation de l'air 22, qui s'échappe du moyen de collecte d'air 10 par ce dernier.

Le débit d'air collecté dans le moyen de collecte d'air 10 peut être par exemple compris entre 20 et 100 litres/minute (l/min). Avantageusement, il est de 50 l/min.

Une fois l'étape de rétention/concentration des microorganismes à l'intérieur de la cartouche réalisée, cette dernière est extraite du moyen de collecte d'air par désolidarisation des éléments supérieur 12 et inférieur 14 dudit moyen, soit par déboîtement, soit par dévissage.

A ce stade, s'offre l'opportunité d'incuber la cartouche dans une étuve à 37°C, si l'on souhaite procéder à la mise en culture des microorganismes concentrés dans ladite cartouche. Comme déjà explicité *supra*, cette incubation dure généralement de quelques dizaines de minutes à quelques heures, pour ne pas allonger de manière trop importante la durée de l'analyse. Néanmoins, si l'obtention d'un résultat d'analyse n'a rien d'urgent, il peut être envisagé d'incuber la cartouche pendant une durée plus longue et plus en adéquation avec les contraintes de la croissance bactérienne, à savoir environ vingt-quatre heures.

De façon similaire, il peut également être envisagé de stocker la cartouche dans un environnement adapté à ce stockage, tel qu'une chambre froide, si l'on souhaite reporter l'analyse.

Selon une variante de l'invention, la cartouche 30 peut être fournie directement placée dans le moyen de collecte de l'air 10. Ce type de présentation a pour avantage d'éviter à l'utilisateur de devoir placer la cartouche dans le moyen de collecte d'air et donc ainsi réduire les risques de contamination lors de cette étape. Il est particulièrement avantageux dans ce cas de figure, que le moyen de collecte d'air 10 soit produit dans le même matériau que la cartouche 30, à savoir en polypropylène, polycarbonate ou PMMA. Une fabrication par moulage par injection est alors particulièrement adaptée.

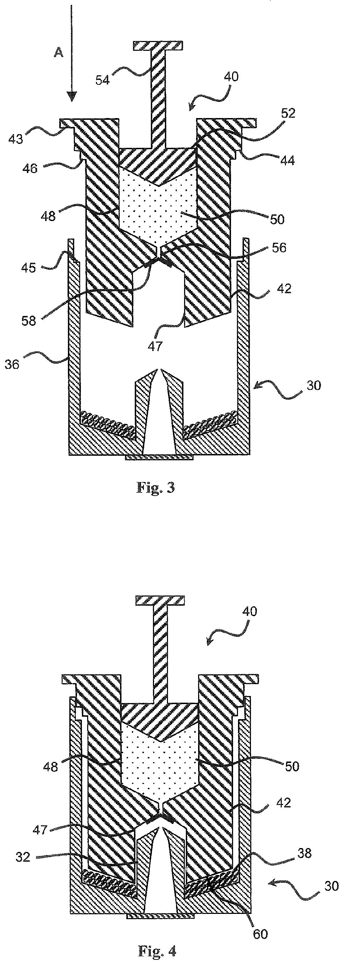

Lorsque l'analyse est réalisée, la cartouche 30 est solidarisée au moyen de récupération des acides nucléiques 40, tel que représenté sur la figure 3. Ledit moyen est inséré dans la cartouche par l'extrémité libre de cette dernière, selon un mouvement translatif vertical représenté par la flèche A.

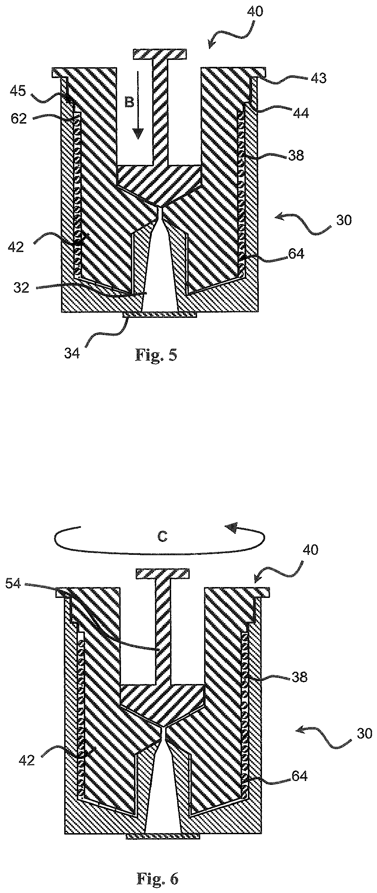

Le moyen de récupération des acides nucléiques 40, représenté en coupe longitudinale sur la figure 3, comporte un corps 42 de forme générale cylindrique à section circulaire. Le corps 42 présente sur la partie supérieure de sa paroi extérieure, un épaulement 43 qui a pour fonction d'assurer l'étanchéité de l'ensemble cartouche 30 - moyen de récupération des acides nucléiques 40, lorsque ce dernier est complètement inséré dans la cartouche 30, en venant en appui sur l'extrémité supérieure de la paroi verticale 36 de la cartouche 30. La paroi extérieure du corps 42 présente par ailleurs plusieurs décrochements successifs dans sa partie haute. Le décrochement 44 situé sous l'épaulement 43, joue lui-même le rôle d'épaulement puisqu'il vient en appui contre l'épaulement 45 réalisé à l'extrémité supérieure de la paroi verticale 36, lorsque le moyen 40 est complètement inséré dans la cartouche 30, tel que représenté sur la figure 5. Le second décrochement 46 positionné sous le décrochement 44 a, quant à lui, pour fonction de permettre la réduction du diamètre du corps 42, de sorte qu'il subsiste un espace interstitiel entre la surface extérieure de la paroi verticale du corps 42 et la surface intérieure de la paroi verticale 36 de la cartouche 30. Le rôle de cet espace sera explicité *infra.* Par ailleurs, l'ensemble de ces épaulements et décrochements permettent de renforcer l'étanchéité de l'ensemble constitué par la cartouche 30 et le moyen de récupération des acides nucléiques 40.

A sa base, le corps 42 présente en position centrale, une cavité 47 de forme sensiblement cylindrique à section circulaire, dont l'extrémité supérieure est de forme sensiblement conique. De même, il comporte également une cavité 48 dans sa partie supérieure, en position centrale. Cette cavité 48, de forme sensiblement cylindrique à section circulaire, dont l'extrémité inférieure est de forme sensiblement conique, constitue une zone de stockage dans laquelle est placé un liquide d'intérêt 50. Ce liquide permet la remise en suspension des billes de verre 38, mais également la récupération des acides nucléiques, une fois les microorganismes lysés. Il est donc important que ce liquide soit inerte vis à vis d'une part des microorganismes et d'autre part, des acides nucléiques, mais également vis à vis des protocoles biologiques. Ainsi , ce liquide d'intérêt est typiquement un tampon adapté à la mise en œuvre de protocoles biologiques. Le volume de liquide d'intérêt contenu dans la cavité 47 peut être compris par exemple entre 0,1 et 0,4 millilitre (ml).

Pour permettre au liquide d'intérêt de jouer son rôle, la cavité 48 coopère avec un moyen d'aspiration/refoulement constitué par un piston 52 dont la forme est complémentaire de la partie inférieure de la cavité 48 et d'un bras 54 en position verticale, solidaire du piston 52, se prolongeant à l'extérieur de la cavité 48. L'extrémité du bras opposée à celle fixée au piston 52, peut présenter une tête de forme particulière apte à coopérer avec un moyen d'actionnement (non représenté) du moyen d'aspiration/refoulement en translation selon un mouvement sensiblement vertical. Ce moyen d'actionnement est préférentiellement un moyen automatisé.

La cavité 48 est en communication fluidique avec la cavité 47 au moyen d'un canal 56, reliant l'extrémité inférieure de la cavité 48 à l'extrémité supérieure de la cavité 47. Sur la figure 3, le canal 56 est obturé au moyen d'une membrane 58 positionnée à l'extrémité supérieure de la cavité 47, empêchant le vidage de la cavité 47. Cette membrane est également dans un matériau apte à être perforé. En particulier, cette membrane peut être perforée au moyen de l'extrémité supérieure du conduit 32 de la cartouche 30.

Comme montré sur la figure 4, les formes extérieures du moyen de récupération des acides nucléiques 40 sont parfaitement complémentaires des formes intérieures de la cartouche 30. Il s'ensuit que le moyen 40 vient parfaitement se positionner à l'intérieur de la cartouche 30, le conduit 32 jouant le rôle de guide en venant s'insérer dans la cavité 47. Le positionnement du moyen 40 se produit jusqu'à ce que la paroi inférieure 60 du moyen 40 se retrouve en appui sur les billes 38.

A ce stade, un mouvement de pression sur le corps 42 du moyen de récupération des acides nucléiques et simultanément sur le moyen de d'aspiration/refoulement conformément à la flèche B représentée sur la figure 5. Ce mouvement translatif du moyen d'aspiration/refoulement entraîne le refoulement du liquide d'intérêt 50 dans la cavité 47 via le canal 56, et plus particulièrement dans l'espace interstitiel entre la paroi interne de la cavité 47 et la paroi externe du conduit 32. Ce refoulement est possible car la pression exercée par le liquide sur la membrane 58, entraîne la perforation ou le décollement de celle-ci. Le liquide d'intérêt 50 chemine alors jusqu'à la zone de rétention de la cartouche où se trouvent les billes de verre 38 et remplit cette zone générant la mise en suspension du matériau gélifié emprisonnant les billes, de sorte que ces dernières sont entraînées dans le flux de liquide d'intérêt, créé par le moyen d'aspiration/refoulement, jusque dans l'espace interstitiel 62, entre le moyen de récupération des acides nucléiques 40 et la cartouche 30. Il est à noter que cet espace interstitiel présente préférentiellement une largeur comprise 600 et 800 µm. Cette largeur est directement liée au diamètre des billes 38 utilisées. En effet, les billes doivent pouvoir circuler facilement dans cet espace mais doivent également pouvoir être mues en rotation par le moyen de récupération des acides nucléiques 40. A cette fin, la paroi verticale externe 64 du corps 42 présente préférentiellement une surface rugueuse, ce qui facilite la mise en rotation des billes.

Une fois toutes les billes positionnées le long de la paroi verticale externe 64 du corps 42, le moyen de récupération des acides nucléiques peut être complètement inséré dans la cartouche, générant ainsi l'étanchéité de l'ensemble, grâce notamment aux épaulements 43 et 44, qui viennent respectivement en appui sur l'extrémité de la paroi de la cartouche 30 et sur l'épaulement 45, tel que représenté sur la figure 5.

Bien entendu, les microorganismes retenus tant sur le matériau gélifié que sur les billes sont transférés dans l'espace interstitiel 62 au même titre que les billes 3, par le flux du liquide d'intérêt.

Concernant le transfert du liquide d'intérêt, il apparaît que la présence de la membrane 34 à la base du conduit 32, empêche l'air contenu dans le conduit 32 d'être évacué lorsque le liquide d'intérêt est poussé dans le canal 56. Il s'ensuit que la pression générée dans le conduit 32 par l'air emprisonné, empêche le liquide d'intérêt de pénétrer à l'intérieur du conduit, qui s'infiltre alors dans l'espace interstitiel entre la paroi interne de la cavité 47 et la paroi externe dudit conduit 32.

Une fois les billes 38 réparties le long de la paroi verticale externe 64 du corps 42 et l'ensemble moyen de récupération des acides nucléiques 40 - cartouche 30 solidarisé, l'étape de lyse proprement dite est initiée. Cette étape est représentée sur la figure 6. Comme on peut le voir sur cette figure, le moyen de récupération des acides nucléiques 40 est mû en rotation, dans le sens représenté par la flèche C. Pour cela, le corps 42 du moyen de récupération des acides nucléiques 40 est solidarisé par tout moyen de couplage mécanique adapté, avec un dispositif automatisé. Un tel système permet en particulier de régler de façon précise la vitesse de rotation.

Il peut néanmoins être envisagé de mettre en rotation le moyen de récupération des acides nucléiques de façon manuelle. Pour cela, des moyens de préhension (non représentés) peuvent être spécifiquement prévus sur la partie supérieure du corps 42. Ces moyens de préhension peuvent alors être saisis d'une main, alors que la cartouche 30 est tenue par l'autre main.

A titre d'exemple, les valeurs de vitesse de rotation peuvent être comprises entre 300 et 2000 tours/min, préférentiellement de 1000 tours/min.

Le temps de rotation est quant à lui généralement compris entre 1 et 2 minutes.

Il est bien évident que le choix de ces deux paramètres est fonction du type de microorganismes que l'on cherche à lyser. Ainsi pour lyser des levures du type *Saccharomyces cerevisiae*, les conditions optimales de lyse consistent dans une vitesse de rotation égale à 1000 tours/min, pendant 2 minutes.

Durant cette étape, les billes 38 sont mises en rotation autour de l'axe de symétrie par frottement contre le corps 42 du moyen de récupération des acides nucléiques. Cette double rotation entraîne la lyse mécanique des microorganismes qui se trouvent pris entre les billes 38 et le corps 42. Il en résulte une libération des acides nucléiques dans le liquide d'intérêt.

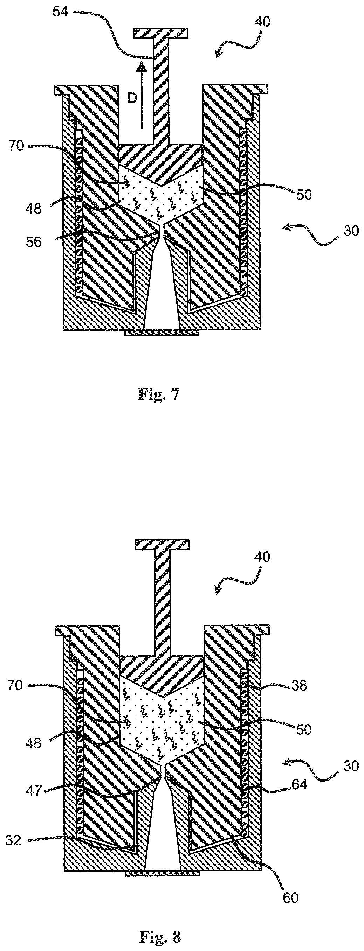

Une fois l'étape de lyse achevée et les acides nucléiques libérés, le liquide d'intérêt est aspiré dans la cavité 48 par le biais du moyen d'aspiration/refoulement, tel que représenté sur la figure 7. De façon détaillée, le moyen d'aspiration /refoulement est mis en mouvement par translation verticale selon la flèche D, par tout moyen approprié automatique ou manuel de traction sur le bras 54. Ce mouvement translatif entraîne l'aspiration du liquide d'intérêt 50 à l'intérieur de la cavité 48. Lors de cette aspiration, le liquide d'intérêt parcourt le chemin inverse de celui qu'il parcourt lors de son refoulement préalablement à l'étape de lyse. En particulier, il progresse dans les espaces interstitiels entre le moyen de récupération des acides nucléiques 40 et la cartouche 30, remonte le canal 56 et aboutit dans la cavité 48. Comme on peut le voir sur les figures 7 et 8, le liquide d'intérêt qui remplit la cavité 48, est chargé en acides nucléiques cibles 70 des microorganismes.

Comme le moyen de récupération des acides nucléiques 40 est entièrement inséré dans la cartouche 30, l'espace interstitiel situé entre la paroi inférieure 60 dudit moyen 40 et le fond de la cartouche, jouant le rôle de zone de rétention des microorganismes libérédes billes n'est pas suffisant pour permettre aux billes 38 de cheminer. Il s'ensuit que les billes 38 restent positionnées le long de la paroi verticale 64 du corps 42.

De même, l'espace interstitiel entre la paroi interne de la cavité 47 et la paroi externe du conduit 32 est particulièrement adapté pour jouer le rôle de filtre, en particulier pour retenir les débris cellulaires produits lors de l'étape de lyse. En effet, lors de la fabrication de la cartouche et du moyen de récupération des acides nucléiques, les dimensions de ces derniers peuvent être adaptées de telle manière que la taille de cet espace interstitiel soit particulièrement bien contrôlée et optimise l'effet de filtrage.

Une fois l'intégralité du liquide d'intérêt aspiré dans la cavité 48, il peut être envisagé de transférer ledit liquide dans un dispositif d'analyse biologique. Ce transfert peut se faire directement dans le dispositif d'analyse ou dans un circuit fluidique dont l'élément final est le dispositif d'analyse.

Lorsque le liquide d'intérêt est transféré directement dans le dispositif, il est envisagé que toutes les étapes de traitement des acides nucléiques permettant l'identification du ou des microorganismes, soient réalisées dans le dispositif lui-même. Ainsi il peut être envisagé par exemple de réaliser dans le dispositif, l'amplification des acides nucléiques cibles, le clivage et le marquage de ces acides nucléiques cibles, et leur détection par hybridation avec des séquences complémentaires.

Sur la figure 9, un dispositif fluidique d'analyse 80 est partiellement représenté en coupe longitudinale. Ce dispositif 80 comporte en sa partie supérieure, une zone préférentielle 82 de connexion fluidique à l'ensemble moyen de récupération des acides nucléiques 40 - cartouche 30. La zone préférentielle de connexion 82 est de forme générale conique, plus particulièrement complémentaire de la forme du conduit 32. Elle présente à son sommet, une ouverture 84 permettant d'accéder au circuit fluidique interne 86 du dispositif 80. Comme représenté sur cette figure 9, le dispositif fluidique d'analyse 80 est connecté à l'ensemble moyen de récupération des acides nucléiques 40 - cartouche 30, par rapprochement de la zone préférentielle de connexion 82 avec la base dudit ensemble, en particulier au niveau de la membrane 34 obturant le conduit 32, conformément à la flèche E. L'extrémité de la zone préférentielle de connexion 82 étant pointue, lorsque celle-ci entre en contact avec la membrane 34, elle perfore cette dernière, libérant l'accès au conduit 32. Le dispositif d'analyse 80 est alors inséré dans le conduit 32 jusqu'à ce qu'il soit en butée, c'est à dire jusqu'à ce que la paroi externe de la zone préférentielle de connexion soit en contact avec la paroi interne du conduit 32, tel que représenté sur la figure 10. Lorsque le dispositif d'analyse 80 est ainsi connecté à l'ensemble moyen de récupération des acides nucléiques 40 - cartouche 30, on constate que l'extrémité de la zone préférentielle de connexion 82 entre en contact avec l'extrémité inférieure du canal 56. Il s'ensuit que la paroi du dispositif fluidique 80, dans la partie supérieure de la zone préférentielle de connexion 82, vient couper la communication fluidique entre le canal 56 et l'espace interstitiel entre la paroi interne de la cavité 47 et la paroi externe du conduit 32.

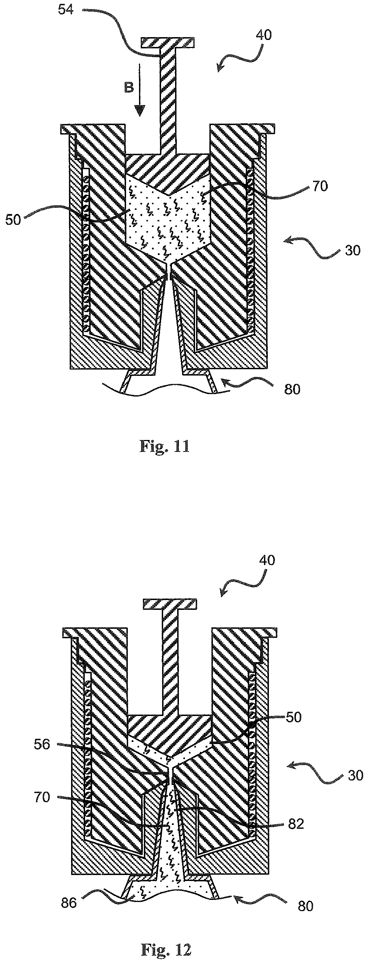

Il s'ensuit que, lorsque l'opérateur souhaite transférer le liquide d'intérêt 50 chargé en acides nucléiques cibles 70 dans le dispositif d'analyse 80, il suffit d'exercer, à nouveau, une pression sur le bras 54 du moyen d'aspiration/refoulement, de sorte que ledit moyen se déplace par translation verticale à l'intérieur de la cavité 48, conformément à la flèche B. Ceci est représenté sur la figure 11.

Le déplacement du moyen d'aspiration/refoulement entraîne le transfert du liquide d'intérêt 50 et des acides nucléiques 70, dans le canal 56 reliant la cavité 48 à la cavité 47. Le canal 56 étant en communication fluidique directe avec le circuit fluidique interne 86 du dispositif fluidique d'analyse 80 par le biais de l'ouverture 84 de la zone préférentielle de connexion 82, le liquide d'intérêt est alors transféré directement dans le dispositif d'analyse 80, sans risque de voir ce dernier entrer en contact avec l'environnement extérieur. Il n'existe pas non plus de risque que le liquide d'intérêt s'échappe dans les espaces interstitiels entre le moyen de récupération des acides nucléiques 40 et la cartouche 30, dans la mesure où toute communication fluidique, entre le canal 56 et l'espace interstitiel entre la paroi interne de la cavité 47 et la paroi externe du conduit 32, est bloquée. Ceci est représenté sur la figure 12.

Une fois l'intégralité du liquide d'intérêt 50 et des acides nucléiques 70 transférés dans le dispositif fluidique d'analyse 80, ce dernier peut être désolidarisé de l'ensemble moyen de récupération des acides nucléiques 40 - cartouche 30. Ces derniers étant usagés, sont jetés. Le dispositif fluidique d'analyse 80 est, quant à lui, ensuite utilisé pour réaliser l'identification des microorganismes.

Selon un mode de réalisation avantageux, toutes les étapes décrites *supra* en relation avec les figures 1 à 12, et en particulier, celles liées à la lyse des microorganismes, l'extraction des acides nucléiques et le transfert desdits acides nucléiques dans le dispositif d'analyse fluidique, peuvent être automatisées au moyen d'un système ad hoc.

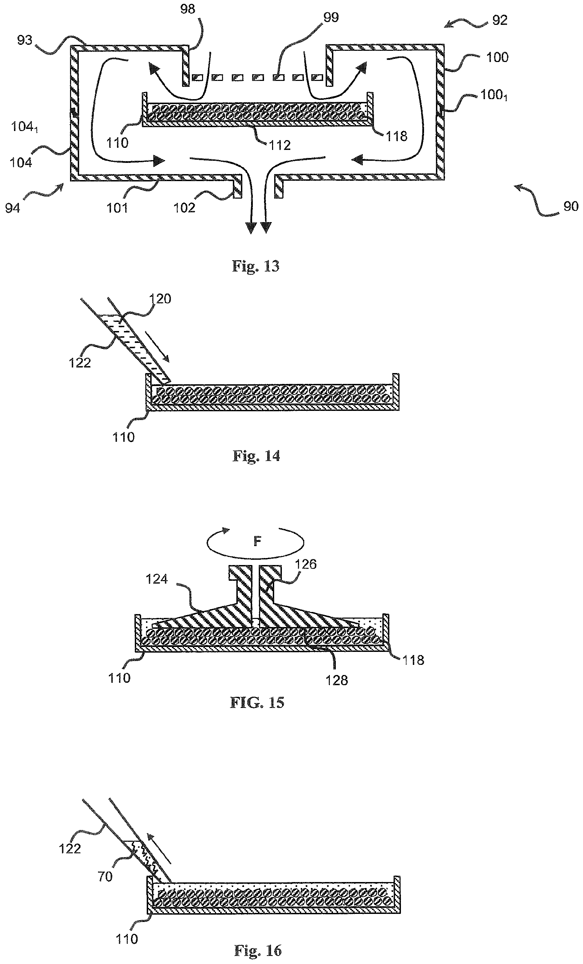

Les figures 13 à 16 représentent un deuxième mode de réalisation du dispositif selon l'invention. Le dispositif considéré est de conception plus simple par rapport au dispositif selon le premier mode de réalisation.

Le dispositif selon ce deuxième mode de réalisation comporte également un moyen de collecte d'air 90. Ce moyen est composé d'un élément supérieur 92 et d'un élément inférieur 94. L'élément supérieur 92 est de forme générale cylindrique. L'extrémité inférieur de ce cylindre est libre, alors que l'extrémité supérieure est partiellement obturée, au moyen d'une paroi horizontale 93. Cette paroi 93 présente en son centre un orifice se prolongeant à l'intérieur de l'élément supérieur 92, par un conduit 98 dont la base est de forme sensiblement conique. Cette partie constitue en fait, le conduit d'admission de l'air à l'intérieur du moyen de collecte d'air 90. Selon un mode de réalisation particulier, ce conduit d'admission d'air peut être connecté à une canalisation d'un circuit de recyclage d'air par tout moyen approprié.

Le conduit 98 comporte en sa partie inférieure une grille 99. Cette grille a pour fonction de permettre une distribution homogène du flux d'air à l'intérieur du moyen de collecte d'air 90.

L'extrémité inférieure de la paroi circulaire verticale 100 de l'élément supérieur 92 comporte, sur sa face extérieure, un épaulement 100₁.Ce décrochement est réalisé sur tout le pourtour de la paroi 100. Il est destiné à faciliter la solidarisation de l'élément supérieur 92 et de l'élément inférieur 94.

L'élément inférieur 94 est également de forme générale cylindrique. L'extrémité supérieure de ce cylindre est libre, alors que l'extrémité inférieure est partiellement obturée, par une paroi horizontale 101 comportant en son centre un conduit d'évacuation de l'air 102, sensiblement cylindrique, la base dudit conduit étant solidaire de la paroi horizontale. L'extrémité inférieure du conduit 102 est libre. Ce conduit est en communication avec l'intérieur de l'élément inférieur 94, de sorte que, quand les éléments supérieur 92 et inférieur 94 du moyen de collecte d'air 90 sont solidarisés, le conduit 102 joue le rôle de conduit d'évacuation de l'air qui a été admis à l'intérieur du moyen de collecte d'air 90, par le biais du conduit d'admission de l'air 98.

L'extrémité supérieure de la paroi circulaire verticale 104 de l'élément inférieur 94 comporte, sur sa face intérieure, un épaulement 104₁.Cet épaulement est réalisé sur tout le pourtour de la paroi 104. Il est également destiné à faciliter la solidarisation de l'élément supérieur 92 et de l'élément inférieur 94, du fait que les extrémités des parois 100 et 104 présentent une section de forme complémentaire, facilitant l'emboîtement. Il importe en premier lieu que cette solidarisation soit réversible. Une fois emboîtés, les éléments 92 et 94 doivent pouvoir se déboîter.

Un moyen de solidarisation alternatif des éléments 92 et 94 peut être une solidarisation par vissage d'un élément sur l'autre. A cette fin, l'extrémité de la paroi d'un des éléments 92 ou 94 peut porter un filetage mâle et l'extrémité de la paroi du deuxième élément un filetage femelle correspondant.

L'important est que le moyen de collecte soit clos de façon hermétique, afin d'éviter toute entrée parasite d'air.

Selon un mode particulier d'utilisation du moyen de collecte de l'air, l'extrémité inférieure du conduit d'évacuation de l'air 102 peut être connectée à une pompe d'aspiration de l'air (non représentée) ou tout moyen de pompage équivalent.

Une cartouche 110 est disposée à l'intérieur du moyen de collecte d'air 90. Pour ce faire, les éléments 92 et 94 constituant le moyen de collecte d'air 90 sont séparés. La cartouche 110 est placée en appui dans l'élément inférieur 94 du moyen de collecte d'air 90. L'élément supérieur 92 est alors repositionné sur l'élément inférieur 94 et ces deux moyens sont solidarisés. L'ensemble constitué par le moyen de collecte d'air 90 et la cartouche 110 est alors, soit connecté à un circuit de recyclage, soit à un dispositif de pompage, afin d'entraîner la circulation de l'air à l'intérieur du moyen de collecte d'air 10, tel que décrit *supra.*

La cartouche 110 représentée en coupe longitudinale, présente une forme générale d'un cylindre de faible hauteur et de section transversale sensiblement circulaire. L'extrémité supérieure du cylindre est libre, alors que l'extrémité inférieure est constituée par une paroi 112. Cette paroi 112 sert de support aux moyens de lyse 118 et constitue la zone de rétention des microorganismes. Ces moyens de lyse sont constitués par des billes, tel que décrit *supra.* Les billes sont maintenues en place sous la forme d'une ou plusieurs couches superposées au moyen d'un matériau gélifié tel que décrit *supra*, déposé sous forme de couche, dans laquelle la ou les couches de billes sont noyées.

Lorsque la circulation d'air dans le moyen de collecte d'air est déclenchée, le chemin suivi par ce dernier est représenté par les flèches sur la figure 13. Ainsi, on constate que l'air entre dans le moyen de collecte d'air 90 par le conduit d'admission de l'air 98. Grâce à la grille 99, il est distribué de manière homogène et vient se briser sur la couche supérieure de billes 118, entraînant à cette endroit la rétention par impaction des microorganismes transportés dans le flux d'air, à la surface du matériau gélifié ou du milieu de culture.

L'air, quant à lui, poursuit sa route aspiré par le moyen de pompage. Il remonte le long de la face interne de la paroi de la cartouche 110. Il redescend le long de la face externe de cette même paroi et se transforme à nouveau en un flux centralisé à l'entrée du conduit d'évacuation de l'air 102, qui s'échappe du moyen de collecte d'air 90 par ce dernier.

Une fois la collecte d'air réalisée, les éléments 92 et 94 du moyen de collecte d'air 90 sont désolidarisés et la cartouche 110 est récupérée.

Intervient ensuite l'étape de distribution du liquide d'intérêt, telle que représentée sur la figure 14. Durant cette étape, le manipulateur vient déposer, dans la cartouche 110, un volume déterminé de liquide d'intérêt 120 à l'aide d'une pipette 122, partiellement représentée sur la figure 14. Comme explicité supra, le liquide d'intérêt peut par exemple être un tampon de lyse. Le contact du liquide d'intérêt avec le matériau gélifié emprisonnant les billes entraine la mise en suspension de ce dernier et donc la mise en suspension des billes elles-mêmes. Le volume de liquide d'intérêt déposé dans la cartouche 110 peut être par exemple de 0,5 ml.

Une fois cette étape réalisée, le manipulateur vient placer dans la cartouche 110 un moyen 124 de récupération des acides nucléiques. Contrairement au moyen 40 décrit dans le premier mode de réalisation, le moyen 124 ne comporte ni moyen d'aspiration/refoulement, ni cavité apte à recevoir le liquide d'intérêt. Ce moyen 124 est de section transversale sensiblement cylindrique, il possède dans sa partie supérieure une zone préférentielle de préhension. 126. La paroi inférieure 128 du moyen 124 est sensiblement plane et vient en appui sur les billes. La lyse est alors mis en œuvre par mise en rotation du moyen 124 selon la flèche F autour de son axe de symétrie, par le manipulateur. La mise en rotation de la paroi 128 entraîne la mise en rotation des billes 118 autour de l'axe de symétrie. Cette double rotation entraîne la lyse mécanique des microorganismes qui se trouvent pris entre les billes 118 et la paroi 128. Il en résulte une libération des acides nucléiques dans le liquide d'intérêt.

Une fois l'étape de lyse achevée et les acides nucléiques libérés, le liquide d'intérêt, chargé desdits acides nucléiques 70, est aspiré par le manipulateur au moyen de la pipette 122, tel que représenté sur la figure 16.

Une fois l'intégralité du liquide d'intérêt dans la pipette, ce dernier peut être transféré dans un dispositif d'analyse afin d'y subir les différentes étapes de traitement des acides nucléiques, telles que décrites *supra.* Il peut également être placé dans un conteneur pour être stocké ou dans tout dispositif destiné à réaliser des étapes préalables aux étapes de traitement des acides nucléiques. Une telle étape peut par exemple être une étape de purification, destinée à concentrer les acides nucléiques en les séparant des constituants cellulaires encore présents. Une telle étape est particulièrement pertinente lorsqu'on utilise le dispositif selon l'invention dans son deuxième mode de réalisation, dans la mesure où, de par la conception simple de ce dernier, le liquide d'intérêt chargé en acides nucléiques, ne subit pas de filtrage, comme c'est le cas dans le dispositif selon le premier mode de réalisation et tel qu'explicité *supra.*

### Exemples:

### Exemple 1 : Mesure de l'efficacité de capture de spores de Bacillus subtilis contenues dans un aérosol par le dispositif selon l'invention

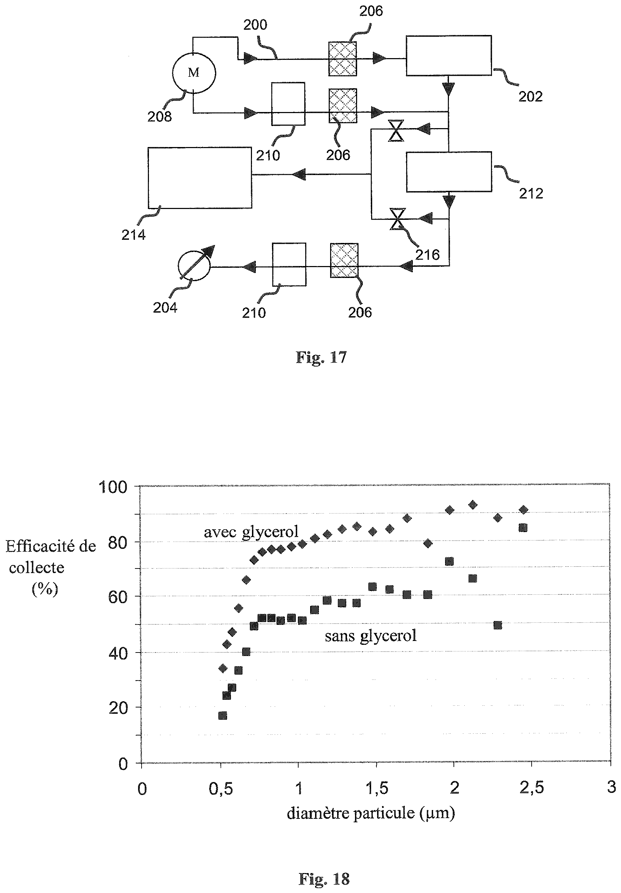

Un banc d'essai étanche a été conçu pour réaliser cette mesure. Ce banc d'essai est représenté sur la figure 17. Il est constitué d'un circuit d'air fermé 200, alimenté en aérosols par un générateur 202 d'aérosols. Ce générateur permet d'introduire dans le flux d'air, une quantité constante de spores de *Bacillus subtilis* (CIP 52.62), à partir d'une suspension de spores de concentration connue. Des générateurs d'aérosols adaptés sont par exemple commercialisés par la société TSI. La circulation de l'air dans le circuit se fait au moyen d'une pompe d'aspiration 204. Trois filtres THE 206 sont présents tout le long du circuit pour permettre l'élimination des particules, en particulier des particules de poussières contenues dans l'air. Un manomètre 208 permet de vérifier la pression dans le circuit et deux débitmètres 210 permettent de vérifier le débit de l'air dans le circuit. Le dispositif selon l'invention est référencé 212. Il est constitué du moyen de collecte d'air 10 à l'intérieur duquel est placée une cartouche 30. Le moyen de collecte est connecté au circuit d'air tel qu'explicité *supra.* Enfin, deux sources de prélèvement, dans le circuit d'air sont positionnées juste en amont et juste en aval du dispositif 212. L'air prélevé à ces niveaux, sous forme de fractions constantes, rejoint un spectromètre 214 permettant de réaliser une mesure aérodynamique des particules, à savoir des spores de *Bacillus subtilis* contenues dans les fractions. Ce spectromètre est un Aerodynamic Particle Sizer® model 3321, commercialisé par la société TSI. Le transfert de l'air dans ce circuit annexe rejoignant le spectromètre est réalisé au moyen de vannes 216, qui permettent d'autoriser ou d'empêcher les prélèvements, soit en amont, soit en aval du dispositif 212. L'analyse différentielle des fractions amont et aval permet de calculer l'efficacité de capture des spores de *Bacillus subtilis* par le dispositif selon l'invention.

Le débit d'air traversant le dispositif selon l'invention est de 50 L/min dans des conditions d'humidité et de température contrôlées (25°C, 35% d'humidité relative). Ce débit est obtenu au moyen de la pompe 204.

L'efficacité de capture du dispositif selon l'invention a été étudiée dans les deux conditions suivantes :
- Détermination de l'efficacité de capture des spores de *Bacillus subtilis*, avec une cartouche dans laquelle les billes 36 sont positionnées dans la cartouche sans matériau gélifié,
- Détermination de l'efficacité de capture des spores de *Bacillus subtilis*, avec une cartouche dans laquelle les billes 36 sont positionnées dans la cartouche et recouvertes d'une fine pellicule de glycérol. Ce produit est suffisamment visqueux pour assurer une bonne adhérence des aérosols à la plaque de collecte et n'interfère généralement pas avec l'analyse moléculaire ultérieure.

Les résultats obtenus avec et sans glycérol sont présentés à la figure 18. Sur cette figure, il est possible de corréler l'efficacité de collecte des spores de *Bacillus subtilis* en fonction du diamètre des particules d'aérosols produits par le générateur 202.

Il apparaît qu'en absence de matériau gélifié recouvrant les billes 36 dans la cartouche, l'efficacité de collecte des spores par le dispositif selon l'invention, peut aller jusqu'à plus de 70%.

En présence de matériau gélifié recouvrant les billes 36 dans la cartouche, l'efficacité de collecte grimpe à plus de 90 %.

L'augmentation de l'efficacité de collecte en présence d'un matériau gélifié, du type glycérol, s'explique par le fait que les spores restent collées sur ce matériau. En absence de matériau gélifié, il se produit un léger phénomène de rebond de spores sur les billes, les empêchant de rester collées.

### Exemple 2 : Détermination de l'efficacité de lyse par comparaison entre la méthode de lyse selon l'invention et des méthodes alternatives.

3 méthodes sont ici comparées à la méthode de lyse selon l'invention :
- Méthode de lyse mécanique mettant en œuvre l'instrument FastPrep™ (type FP120, BIO101, commercialisé par la société Thermo Electron Corporation);
- Méthode de lyse enzymatique, utilisant du lysozyme ;
- Méthode utilisant un agent chaotropique (isothiocyanate de gaunidium, GuSCN) combinée à une étape de chauffage de la suspension de cellules à 90°C, suivi de trois étapes de congélation-décongélation des cellules.

Dans cet exemple, les cellules utilisées sont des *Bacillus cereus* en suspension.

Après l'étape de lyse, les acides nucléiques récupérés sont purifiés en utilisant le système d'extraction NucliSENS® easyMAG™ commercialisée par la demanderesse. Les acides nucléiques purifiés sont enfin quantifiés en utilisant la méthode de détection SYBRGreen (Molecular Probes). Les étapes précises de l'étude comparative sont décrites ci-dessous :

### - Etape 1 : Préparation de la suspension de Bacillus cereus

Une culture de *Bacillus cereus* est réalisée sur milieu de culture gélosé en boite TSA (trypticase soja). Des colonies bactériennes sont récupérées sur la gélose et lavées dans un tampon d'extraction NucliSENS® easyMAG™ Extraction Buffer 3 (réf. 280132), commercialisé par la demanderesse. Une fois les cellules lavées, elles sont diluées dans 5 ml du même tampon d'extraction, afin d'obtenir une suspension bactérienne ayant une densité optique de 0,5 à une longueur d'ondes de 600 nm.

### - Etape 2a : Lyse avec l'instrument FastPrep®

0,5 ml de la suspension bactérienne est transféré dans un tube de 2 ml rempli avec un mélange de 1g de billes de verre (fractions égales de billes de différentes tailles : 150-212 µm, 420-600 µm et 700-1180 µm). L'instrument FastPrep® est utilisé à sa vitesse maximum (réglage à 6,0), pendant 60 sec. Une fois la lyse achevée, 100 µl de lysat sont transférés dans l'instrument NucliSENS® easyMAG™ pour subir une étape de purification.

### - Etape 2b : Lyse avec le dispositif selon l'invention

0,3 ml de suspension bactérienne est transférée dans une cartouche équivalente à la cartouche 110 décrite *supra.* La cartouche est ensuite remplie avec 0,4 g de mélanges de billes de verre (fractions égales de billes de différentes tailles : 150-212 µm, 212-300 µm et 420-600 µm). La cartouche est mise en contact avec un moyen de récupération des acides nucléiques. Ce dernier est mis en rotation pendant 2 min. à 2000 tours/min. Une fois cette étape de lyse réalisée, 100 µl de lysat sont transférés dans l'instrument NucliSENS® easyMAG™ pour subir une étape de purification.

### - Etape 2c : Lyse enzymatique

Dans cette méthode de lyse avec lysozyme, 100 µl de suspension bactérienne sont mélangés à 100 µl d'une solution de lysozyme (50 mg/ml). Le mélange est incubé 30 min. à 37°C. Après cette incubation, l'intégralité du lysat (200 µl) est transférée dans l'instrument NucliSENS® easyMAG™ pour subir une étape de purification.

### - Etape 2d : Lyse chaotropique

100 µl de suspension bactérienne sont mélangés à 100 µl d'une solution concentrée de GuSCN (5 moles/l) et le tout est incubé pendant 30 min. Après cette incubation, l'intégralité du lysat (200 µl) est transférée dans l'instrument NucliSENS® easyMAG™ pour subir une étape de purification.

### - Etape 3 : Purification du lysat à l'aide de l'instrument NucliSENS® easyMAG™

L'étape de purification est réalisée en utilisant le protocole d'extraction générique de l'instrument NucliSENS® easyMAG™. On ajoute à chaque lysat, 2 ml de tampon de lyse NucliSENS® easyMAG™ Lysis Buffer (ref. 280134) commercialisé par la demanderesse et 50 µl de d'une suspension de particules de silice magnétiques NucliSENS® easyMAG™ Magnetic Silica (Ref. 280133) commercialisée par la demanderesse. Les acides nucléiques de chacun des lysats sont élués dans 25 µl de tampon d'extraction NucliSENS® easyMAG™ Extraction Buffer 3.

### - Etape 4 : Détection des acides nucléiques

Pour déterminer la quantité d'acides nucléiques dans chacun des éluats, 10 µl desdits éluats dans un tube PCR de 0,2 ml. 180 µl de solution SyberGreen sont ensuite ajoutés aux échantillons et le signal de fluorescence est mesuré en utilisant l'instrument NucliSENS EasyQ® commercialisé par la demanderesse.

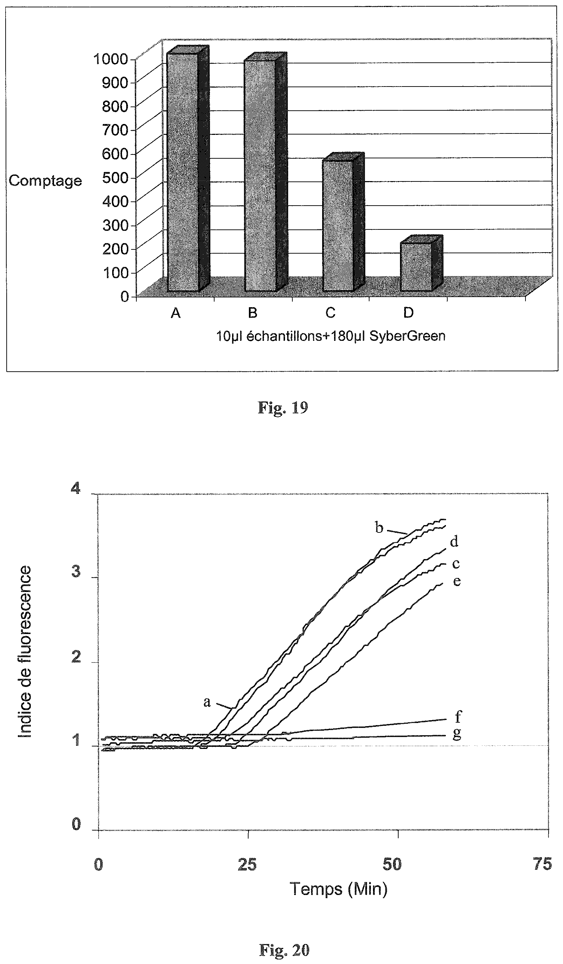

Les résultats des mesures de fluorescence sont présentés sur la figure 19. La correspondance est la suivante :
A = Lyse avec l'instrument FastPrep®
B = Lyse avec le dispositif selon l'invention
C = Lyse enzymatique
D = Lyse chaotropique

En analysant la figure 19, on constate que l'efficacité de lyse du dispositif selon l'invention est similaire est celle de la méthode de lyse mécanique FastPrep®. Ces deux méthodes mécaniques sont par ailleurs significativement plus efficaces que la méthode de lyse enzymatique ou de lyse chimique utilisant l'agent chaotropique.

### Exemple 3 : Lyse d'une suspension de Bacillus à l'aide du dispositif selon l'invention en combinaison avec une détection par la méthode NASBA

0,3 ml d'une suspension de *Bacillus cereus* dilué dans le tampon d'extraction NucliSENS® easyMAG™ Extraction Buffer 3 est introduit dans la cartouche selon l'invention. Le procédé de lyse selon l'invention, tel que décrit dans l'exemple précédent est reproduit pendant 2 min. Après l'étape de lyse, 5 µl de lysat sont utilisés pour réaliser une détection par la méthode NASBA en temps réel (Real Time NASBA) en utilisant des amorces et sondes spécifiques de *Bacillus cereus*, par fluorescence à l'aide de l'instrument NucliSENS EasyQ®. Le gène cible utilisé pour la détection est le gène EF-tu. La taille du produit d'amplification est de 115 nucléotides.

Différentes quantités de *Bacillus* allant de 6 à 6.10⁵ ont été utilisées.

La figure 20 montre la production de fluorescence en temps réel et en fonction des différentes quantités de *Bacillus cereus* introduites dans le dispositif selon l'invention. Sur cette figure, la correspondance est la suivante :
a = Echantillon avec 6.10⁵ *Bacilli*
b = Echantillon avec 6.10⁴ *Bacilli*
c = Echantillon avec 6.10³ *Bacilli*
d = Echantillon avec 600 *Bacilli*
e = Echantillon avec 60 *Bacilli*
f = Echantillon avec 6 *Bacilli*
g = Echantillon sans Bacillus

Au regard de la figure 20, on observe une très bonne corrélation entre le niveau de fluorescence détecté et le nombre de bactéries contenues dans l'échantillon. De plus, on constate une différence significative entre un échantillon vierge de bactérie et un échantillon n'en contenant que 6. Enfin, on peut déduire des résultats de cette analyse que le dispositif selon l'invention offre un niveau de performance en adéquation avec l'utilisation d'une méthode d'amplification des acides nucléiques du type NASBA.

Il est à noter que la sensibilité de cette analyse pourrait être améliorée en concentrant les acides nucléiques, avant de réaliser l'amplification par la méthode NASBA. Une telle étape de concentration peut être réalisée en utilisant par exemple l'instrument NucliSENS® easyMAG™.

### Exemple 4 : Analyse d'un échantillon de sang total, utilisant le dispositif selon l'invention

Dans cet exemple, le dispositif selon l'invention est utilisé pour analyser un échantillon de sang total, dans lequel une population de *Bacillus cereus* a été ajoutée. L'échantillon de sang est issu d'une personne saine.

Une culture de *Bacillus cereus* est réalisée sur milieu de culture gélosé en boite TSA (trypticase soja). Des colonies bactériennes sont récupérées sur la gélose et lavées dans un tampon d'extraction NucliSENS® easyMAG™ Extraction Buffer 3. Une fois les cellules lavées, elles sont diluées dans 5 ml du même tampon d'extraction, afin d'obtenir une suspension bactérienne ayant une densité optique de 0,5 à une longueur d'ondes de 600 nm. Cette suspension mère comporte environ 2,2. 10⁷ bactéries/ml. Cette suspension mère est diluée dans du tampon d'extraction NucliSENS® easyMAG™ Extraction Buffer 3, afin d'obtenir de suspension de concentrations différentes, une à 10³ bactéries/ml et une à 10⁵ bactéries/ml.

Le protocole ensuite utilisé est le suivant pour chacune des suspensions filles :
1. 24 µl de suspension fille sont ajoutés à 150 µl de sang et mélangés ;
2. 125µl d'un tampon d'extraction NucliSENS® easyMAG™ Extraction Buffer 1 (ref. 280130) sont introduits dans une cartouche selon l'invention ;
3. l'échantillon de sang chargé en bactéries est à son tour introduit dans la cartouche ;
4. l'étape de lyse est réalisée en mettant la cartouche en contact avec un moyen de récupération des acides nucléiques. Ce dernier est mis en rotation pendant 2 min. à 2000 tours/min ;
5. 100 µl de lysat sont aspirés dans la cartouche ;
6. une purification de ce lysat est réalisée à l'aide de l'instrument NucliSENS® easyMAG™ tel que décrit à l'étape 3 de l'exemple 2, permettant de récupérer 25 µl d'éluat ;
7. 5 µl de cet éluat sont utilisés pour effectuer l'amplification et la détection, par la technologie Real Time NASBA

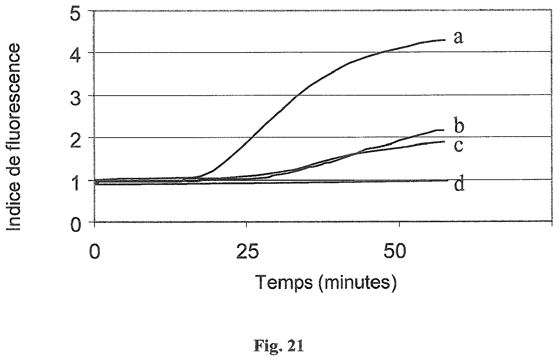

Les résultats sont présentés à la figure 21. Sur cette figure, la correspondance est la suivante :
a = Echantillon de sang total contenant 53 *Bacilli* (suspension fille à 10⁵ bactéries/ml)
b = Echantillon de sang total contenant 5 *Bacilli* (suspension fille à 10³ bactéries/ml)
c = Echantillon de sang total contenant 5 *Bacilli* (suspension fille à 10³ bactéries/ml)
d = Echantillon de sang total sans bactéries

Les résultats présentés correspondent aux suspensions filles à 10³ et 10⁵ bactéries/ml. Par ailleurs, l'analyse pour la suspension à 10⁵ bactéries/ml a été dupliquée.

Au regard du volume de suspensions filles introduit dans la cartouche selon l'invention, les quantités effectives de bactéries sont de 5 et 530 bactéries, respectivement pour les suspensions à 10³ et 10⁵ bactéries/ml.

On constate ainsi que l'analyse d'un échantillon ne contenant que 5 bactéries avec le dispositif selon l'invention produit un signal positif de détection (courbes b et c), se différenciant nettement du résultat obtenu avec l'échantillon vierge de bactéries (courbe d). On constate par ailleurs, que même avec des quantités faibles de bactéries, les résultats sont reproductibles ; c'est ce que montrent les courbes b et c.

Il est à noter que cette très bonne sensibilité n'a été obtenue qu'en utilisant que 5 µl des 25 µl obtenus à l'issu de l'étape de purification. Cette sensibilité peut donc être encore améliorée en réalisant une étape de concentration de l'échantillon, après purification.

Il ressort ainsi de ces exemples que le dispositif selon l'invention est un outil performant pour collecter les bactéries contenues dans un échantillon d'air, les lyser et permettre la récupération des acides nucléiques desdites bactéries à fins d'analyse. Pour cela, le dispositif mis en œuvre sera constitué dans un premier temps d'une cartouche insérée à l'intérieur du moyen de collecte d'air et, dans un deuxième temps, de la cartouche contenant les bactéries collectées, combinée au moyen de récupération des acides nucléiques.

Le dispositif selon l'invention est également tout à fait adapté pour l'analyse d'échantillons cliniques, tels que des échantillons de sang total. Dans ce cas, la cartouche est utilisée sans moyen de collecte d'air. L'échantillon liquide est placé dans la cartouche puis celle-ci est combinée au moyen de récupération des acides nucléiques afin d'assurer la lyse des bactéries et la récupération des acides nucléiques.

## Revendications

1. Cartouche, destinée à être positionnée à l'intérieur d'un moyen de collecte d'air et à recevoir un moyen de récupération des acides nucléiques, ladite cartouche de forme sensiblement cylindrique, comportant une zone de rétention des microorganismes, ladite zone de rétention comportant des moyens de lyse de microorganismes, constitués par des billes, et un matériau gélifié, inerte, apte à retenir les microorganismes, à maintenir lesdits moyens de lyse en place et à se dissoudre en présence d'un liquide.

2. Cartouche selon la revendication précédente, dans lequel le matériau gélifié est un milieu de culture des microorganismes

3. Cartouche selon l'une des revendications précédentes, comportant en outre un moyen de connexion d'un dispositif d'analyse.

4. Cartouche selon l'une des revendications précédentes, dans laquelle le diamètre des billes est compris entre 200 et 600 µm.

5. Dispositif de collecte de microorganismes contenus dans l'air, ledit dispositif comportant :
- un moyen de collecte d'air, comprenant un élément supérieur comportant un conduit d'admission de l'air et un élément inférieur comportant un conduit d'évacuation de l'air, lesdits éléments supérieur et inférieur pouvant être solidarisés l'un à l'autre de sorte qu'un courant d'air puisse être créé à l'intérieur dudit moyen de collecte d'air ;
- une cartouche selon l'une des revendications 1 à 4, positionnée à l'intérieur dudit moyen de collecte d'air.

6. Dispositif selon la revendication précédente, dans le moyen de collecte d'air est apte à être connecté à un circuit de recyclage de l'air.

7. Dispositif de lyse de microorganismes, dans le but d'isoler les acides nucléiques desdits microorganismes, ledit dispositif comportant :
- une cartouche selon l'une des revendications 1 à 4, ladite cartouche comportant des microorganismes disposés dans la zone de rétention des microorganismes ;
- un moyen de récupération des acides nucléiques, de forme sensiblement cylindrique, apte à être placé dans la cartouche, ledit moyen de récupération, coopérant avec les moyens de lyse des microorganismes afin de lyser lesdits microorganismes et permettre la libération des acides nucléiques.

8. Dispositif selon la revendication précédente, dans lequel le moyen de récupération des acides nucléiques comporte un moyen d'aspiration/refoulement de liquide.

9. Dispositif selon l'une des revendications 7 ou 8, dans lequel les moyens de récupération des acides nucléiques comportent, en outre, une zone de stockage d'un liquide.

10. Dispositif selon l'une des revendications 7 à 9, dans lequel le diamètre interne de la cartouche est supérieur au diamètre externe du moyen de récupération des acides nucléiques, de sorte que lorsque le moyen de récupération des acides nucléiques est emboîté dans la cartouche, la distance séparant la paroi interne de la cartouche de la paroi externe du moyen de récupération des acides nucléiques soit suffisamment importante pour permettre aux moyens de lyse de venir se positionner dans cet espace interstitiel et suffisamment faible pour que les moyens de lyse soient au contact de l'une ou l'autre desdites parois.

11. Procédé de concentration des microorganismes contenus dans l'air, ledit procédé comprenant les étapes consistant à :
a) Placer une cartouche selon l'une des revendications 1 à 4, à l'intérieur du moyen de collecte d'air, de sorte que la zone de rétention, à l'intérieur de ladite cartouche, soit en communication avec le conduit d'admission d'air du moyen de collecte d'air,
b) Provoquer l'entrée d'air à l'intérieur dudit moyen de collecte d'air par tout moyen approprié,
c) Concentrer les microorganismes contenus dans l'air dans la zone de rétention de la cartouche.

12. Procédé de concentration selon la revendication précédente, qui comporte en outre une étape d) consistant faire croître les microorganismes dans la zone de rétention.

13. Procédé de concentration selon l'une des revendications 11 ou 12 dans lequel, les microorganismes sont retenus sur les moyens de lyse, présents dans la zone de rétention.

14. Procédé de lyse de microorganismes contenus dans l'air, ledit procédé comportant les étapes consistant à :
a) Placer une cartouche, selon l'une des revendications 1 à 4, à l'intérieur du moyen de collecte d'air, de sorte que la zone de rétention, à l'intérieur de ladite cartouche, soit en communication avec le conduit d'admission d'air du moyen de collecte d'air,
b) Provoquer l'entrée d'air à l'intérieur dudit moyen de collecte d'air par tout moyen approprié,
c) Concentrer les microorganismes contenus dans l'air, dans la zone de rétention de la cartouche,
d) Sortir la cartouche du moyen de collecte d'air,
e) Placer le moyen de récupération des acides nucléiques dans la cartouche,
f) Introduire un liquide d'intérêt dans la cartouche, entraînant la mise en suspension des moyens de lyse situés dans la zone de rétention des microorganismes de la cartouche, et
g) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes.

15. Procédé de lyse de microorganismes contenus dans l'air, ledit procédé comportant les étapes consistant à :
a) Placer une cartouche, selon l'une des revendications 1 à 4, à l'intérieur du moyen de collecte d'air, de sorte que la zone de rétention, à l'intérieur de ladite cartouche, soit en communication avec le conduit d'admission d'air du moyen de collecte d'air,
b) Provoquer l'entrée d'air à l'intérieur dudit moyen de collecte d'air par tout moyen approprié,
c) Concentrer les microorganismes contenus dans l'air, dans la zone de rétention de la cartouche,
d) Sortir la cartouche du moyen de collecte d'air,
e) Placer le moyen de récupération des acides nucléiques dans la cartouche par emboîtement,
f) Provoquer le refoulement d'un liquide d'intérêt placé préalablement dans la zone de stockage du moyen de récupération des acides nucléiques, ledit refoulement étant obtenu par le biais du moyen d'aspiration/refoulement du moyen de récupération des acides nucléiques, le liquide ainsi refoulé venant remplir l'espace interstitiel situé entre le moyen de récupération des acides nucléiques et la cartouche, entraînant la mise en suspension des moyens de lyse situés dans la zone de rétention des microorganismes de la cartouche, lesdits moyens de lyse venant se positionner entre la paroi intérieure verticale de la cartouche et la paroi extérieure verticale du moyen de récupération des acides nucléiques, et
g) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes.

16. Procédé d'extraction des acides nucléiques de microorganismes contenus dans l'air, ledit procédé comportant les étapes consistant à :
a) Placer une cartouche, selon l'une des revendications 1 à 4, à l'intérieur du moyen de collecte d'air, de sorte que la zone de rétention, à l'intérieur de ladite cartouche, soit en communication avec le conduit d'admission d'air du moyen de collecte d'air,
b) Provoquer l'entrée d'air à l'intérieur dudit moyen de collecte d'air par tout moyen approprié,
c) Concentrer les microorganismes contenus dans l'air, dans la zone de rétention de la cartouche,
d) Sortir la cartouche du moyen de collecte d'air,
e) Placer le moyen de récupération des acides nucléiques dans la cartouche,
f) Introduire un liquide d'intérêt dans la cartouche, entraînant la mise en suspension des moyens de lyse situés dans la zone de rétention des microorganismes de la cartouche, et
g) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes, et
h) Aspirer le liquide d'intérêt contenant les acides nucléiques desdits microorganismes libérés lors de la lyse.

17. Procédé d'extraction des acides nucléiques de microorganismes contenus dans l'air, ledit procédé comportant les étapes consistant à :
a) Placer une cartouche, selon l'une des revendications 1 à 4, à l'intérieur du moyen de collecte d'air, de sorte que la zone de rétention, à l'intérieur de ladite cartouche, soit en communication avec le conduit d'admission d'air du moyen de collecte d'air,
b) Provoquer l'entrée d'air à l'intérieur dudit moyen de collecte d'air par tout moyen approprié,
c) Concentrer les microorganismes contenus dans l'air, dans la zone de rétention de la cartouche,
d) Sortir la cartouche du moyen de collecte d'air,
e) Placer le moyen de récupération des acides nucléiques dans la cartouche par emboîtement,
f) Provoquer le refoulement d'un liquide d'intérêt placé préalablement dans la zone de stockage du moyen de récupération des acides nucléiques, ledit refoulement étant obtenu par le biais du moyen d'aspiration/refoulement du moyen de récupération des acides nucléiques, le liquide ainsi refoulé venant remplir l'espace interstitiel situé entre le moyen de récupération des acides nucléiques et la cartouche, entraînant la mise en suspension des moyens de lyse situés dans la zone de rétention des microorganismes de la cartouche, lesdits moyens de lyse venant se positionner entre la paroi intérieure verticale de la cartouche et la paroi extérieure verticale du moyen de récupération des acides nucléiques,
g) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes, libérant ainsi les acides nucléiques desdits microorganismes, et
h) Provoquer l'aspiration du liquide d'intérêt dans la zone de stockage du moyen de récupération des acides nucléiques, ladite aspiration étant obtenue par le biais du moyen d'aspiration/refoulement du moyen de récupération des acides nucléiques, le liquide d'intérêt ainsi aspiré contenant les acides nucléiques desdits microorganismes libérés lors de la lyse.

18. Procédé selon l'une des revendications 14 à 17, comportant une étape supplémentaire d') consistant à faire croître les microorganismes concentrés dans la zone de rétention de la cartouche.

19. Procédé selon la revendication précédente, dans lequel la mise en croissance est obtenue par incubation de la cartouche dans une étuve pendant un temps allant de 2 à 24 heures.

20. Procédé de lyse de microorganismes, ledit procédé comportant les étapes consistant à :
a) Obtenir une cartouche, selon l'une des revendications 1 à 4, dans laquelle des microorganismes sont concentrés dans la zone de rétention,
b) Placer le moyen de récupération des acides nucléiques dans la cartouche par emboîtement,
c) Provoquer le refoulement d'un liquide d'intérêt placé préalablement dans la zone de stockage du moyen de récupération des acides nucléiques, ledit refoulement étant obtenu par le biais du moyen d'aspiration/refoulement du moyen de récupération des acides nucléiques, le liquide ainsi refoulé venant remplir l'espace interstitiel situé entre le moyen de récupération des acides nucléiques et la cartouche, entraînant la mise en suspension des moyens de lyse situés dans la zone de rétention des microorganismes de la cartouche, lesdits moyens de lyse venant se positionner entre la paroi intérieure verticale de la cartouche et la paroi extérieure verticale du moyen de récupération des acides nucléiques, et
d) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes.

21. Procédé de lyse de microorganismes, ledit procédé comportant les étapes consistant à :
a) Obtenir une cartouche, selon l'une des revendications 1 à 4, dans laquelle des microorganismes sont concentrés dans la zone de rétention,
b) Placer le moyen de récupération des acides nucléiques dans la cartouche,
c) Introduire un liquide d'intérêt dans la cartouche, entraînant la mise en suspension des moyens de lyse situés dans la zone de rétention des microorganismes de la cartouche, et
d) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes.

22. Procédé d'extraction des acides nucléiques de microorganismes, ledit procédé comportant les étapes consistant à :
a) Obtenir une cartouche, selon l'une des revendications 1 à 4, dans laquelle des microorganismes sont concentrés dans la zone de rétention,
b) Placer le moyen de récupération des acides nucléiques dans la cartouche par emboîtement,
c) Provoquer le refoulement d'un liquide d'intérêt placé préalablement dans la zone de stockage du moyen de récupération des acides nucléiques, ledit refoulement étant obtenu par le biais du moyen d'aspiration/refoulement du moyen de récupération des acides nucléiques, le liquide ainsi refoulé venant remplir l'espace interstitiel situé entre le moyen de récupération des acides nucléiques et la cartouche, entraînant la mise en suspension des moyens de lyse situés dans la zone de rétention des microorganismes de la cartouche, lesdits moyens de lyse venant se positionner entre la paroi intérieure verticale de la cartouche et la paroi extérieure verticale du moyen de récupération des acides nucléiques,
d) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes, libérant ainsi les acides nucléiques desdits microorganismes, et
e) Provoquer l'aspiration du liquide d'intérêt dans la zone de stockage du moyen de récupération des acides nucléiques, ladite aspiration étant obtenue par le biais du moyen d'aspiration/refoulement du moyen de récupération des acides nucléiques, le liquide d'intérêt ainsi aspiré contenant les acides nucléiques desdits microorganismes libérés lors de la lyse.

23. Procédé d'extraction des acides nucléiques de microorganismes, ledit procédé comportant les étapes consistant à :
a) Obtenir, selon l'une des revendications 1 à 4, une cartouche dans laquelle des microorganismes sont concentrés dans la zone de rétention,
b) Placer le moyen de récupération des acides nucléiques dans la cartouche,
c) Introduire un liquide d'intérêt dans la cartouche, entraînant la mise en suspension des moyens de lyse situés dans la zone de rétention des microorganismes de la cartouche, et
d) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes, et
e) Aspirer le liquide d'intérêt contenant les acides nucléiques desdits microorganismes libérés lors de la lyse.

24. Procédé de lyse de microorganismes, ledit procédé comportant les étapes consistant à :
a) Introduire un échantillon liquide contenant lesdits microorganismes dans une cartouche selon l'une des revendications 1 à 4, au voisinage de la zone de rétention, de sorte que ledit échantillon liquide entraîne la mise en suspension des moyens de lyse situés dans ladite zone de rétention des microorganismes de la cartouche,
b) Placer le moyen de récupération des acides nucléiques dans la cartouche,
c) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes.

25. Procédé d'extraction des acides nucléiques de microorganismes, ledit procédé comportant les étapes consistant à :
a) Introduire un échantillon liquide contenant lesdits microorganismes dans une cartouche selon l'une des revendications 1 à 4 au voisinage de la zone de rétention, de sorte que ledit échantillon liquide entraîne la mise en suspension des moyens de lyse situés dans ladite zone de rétention des microorganismes de la cartouche,
b) Placer le moyen de récupération des acides nucléiques dans la cartouche,
c) Lyser mécaniquement les microorganismes, par mise en rotation du moyen de récupération des acides nucléiques, à l'intérieur de la cartouche, ledit moyen de récupération des acides nucléiques entraînant en rotation les moyens de lyse sur lesquels sont retenus les microorganismes,
d) Aspirer le liquide d'intérêt contenant les acides nucléiques desdits microorganismes libérés lors de la lyse.

26. Procédé d'identification d'un ou plusieurs microorganismes contenus dans un prélèvement, comprenant les étapes consistant à
- Isoler les acides nucléiques des microorganismes contenus dans ledit prélèvement au moyen du dispositif selon l'une des revendications 5 à 10,
- Identifier le ou les microorganismes ainsi isolés.

27. Procédé d'identification selon la revendication précédente, comportant en outre une étape intermédiaire consistant à purifier les acides nucléiques.

28. Procédé d'identification selon l'une des revendications 26 ou 27, dans lequel l'étape d'identification comporte les sous-étapes consistant à :
- amplifier de façon spécifique les acides nucléiques isolés,
- détecter les acides nucléiques ainsi amplifiés.

29. Procédé d'identification selon l'une des revendications 26 à 28, dans lequel l'étape d'identification est réalisée dans un dispositif d'identification, en communication fluidique avec la cartouche du dispositif selon l'une des revendications 7 à 10.

30. Procédé d'identification selon la revendication précédente, dans lequel les acides nucléiques isolés sont transférés du moyen de récupération des acides nucléiques du dispositif selon l'une des revendications 7 à 10, vers le dispositif d'identification.

31. Procédé d'identification selon la revendication précédente, dans lequel le transfert des acides nucléiques est obtenu par refoulement du liquide d'intérêt contenant les acides nucléiques, ledit liquide d'intérêt étant contenu dans la zone de stockage du moyen de récupération des acides nucléiques, par le biais du moyen d'aspiration/refoulement du moyen de récupération des acides nucléiques.

## Patentansprüche

1. Kartusche zum Positionieren im Inneren eines Luftsammelmittels und zum Aufnehmen eines Nukleinsäuregewinnungsmittels, wobei die Kartusche mit einer im Wesentlichen zylindrischen Form eine Mikroorganismenrückhaltezone umfasst, wobei die Rückhaltezone Mikroorganismenlysemittel umfasst, die aus Kugeln und einem inerten gelierten Material bestehen, das fähig ist, die Mikroorganismen zurückzuhalten, die Lysemittel an Ort und Stelle zu halten und sich in Gegenwart einer Flüssigkeit zu lösen.

2. Kartusche nach dem vorhergehenden Anspruch, wobei das gelierte Material ein Kulturmedium für Mikroorganismen ist.

3. Kartusche nach einem der vorhergehenden Ansprüche, die ferner ein Mittel zum Verbinden mit einer Analysevorrichtung umfasst.

4. Kartusche nach einem der vorhergehenden Ansprüche, wobei der Durchmesser der Kugeln zwischen 200 und 600 µm beträgt.

5. Vorrichtung zum Sammeln von in der Luft enthaltenen Mikroorganismen, wobei die Vorrichtung umfasst:
- ein Luftsammelmittel, umfassend ein oberes Element, das einen Lufteinlasskanal umfasst, und ein unteres Element, das einen Luftauslasskanal umfasst, wobei das obere und das untere Element miteinander derart verbunden werden können, dass ein Luftstrom im Inneren des Luftsammelmittels erzeugt werden kann;
- eine Kartusche nach einem der Ansprüche 1 bis 4, die im Inneren des Luftsammelmittels positioniert ist.

6. Vorrichtung nach dem vorhergehenden Anspruch, wobei das Luftsammelmittel mit einem Luftrezirkulationskreislauf verbunden werden kann.

7. Mikroorganismenlysevorrichtung zum Isolieren der Nukleinsäuren der Mikroorganismen, wobei die Vorrichtung umfasst:
- eine Kartusche nach einem der Ansprüche 1 bis 4, wobei die Kartusche Mikroorganismen umfasst, die in der Mikroorganismenrückhaltezone angeordnet sind;
- ein Nukleinsäuregewinnungsmittel mit im Wesentlichen zylindrischer Form, das in der Kartusche platziert werden kann, wobei das Gewinnungsmittel mit den Mikroorganismenlysemitteln zusammenwirkt, um die Mikroorganismen zu lysieren und die Freisetzung der Nukleinsäuren zu ermöglichen.

8. Vorrichtung nach dem vorhergehenden Anspruch, wobei das Nukleinsäuregewinnungsmittel ein Flüssigkeitsansaug-/-pumpmittel umfasst.

9. Vorrichtung nach einem der Ansprüche 7 oder 8, wobei die Nukleinsäuregewinnungsmittel ferner eine Flüssigkeitsaufbewahrungzone umfassen.

10. Vorrichtung nach einem der Ansprüche 7 bis 9, wobei der Innendurchmesser der Kartusche größer ist als der Außendurchmesser des Nukleinsäuregewinnungsmittels, so dass, wenn das Nukleinsäuregewinnungsmittel in die Kartusche eingesteckt ist, der Abstand, der die Innenwand der Kartusche von der Außenwand des Nukleinsäuregewinnungsmittels trennt, ausreichend groß ist, um zu ermöglichen, dass die Lysemittel sich in diesem Zwischenraum positionieren, und ausreichend klein ist, dass die Lysemittel mit der einen oder anderen der Wände in Kontakt stehen.

11. Verfahren zum Aufkonzentrieren von in der Luft enthaltenen Mikroorganismen, wobei das Verfahren die Schritte umfasst:
a) Platzieren einer Kartusche nach einem der Ansprüche 1 bis 4 im Inneren des Luftsammelmittels, so dass die Rückhaltezone im Inneren der Kartusche mit dem Lufteinlasskanal des Luftsammelmittels in Kommunikation steht,
b) Herbeiführen von Lufteintritt in das Innere des Luftsammelmittels durch jedes geeignete Mittel,
c) Aufkonzentrieren der in der Luft enthaltenen Mikroorganismen in der Rückhaltezone der Kartusche.

12. Aufkonzentrierungsverfahren nach dem vorhergehenden Anspruch, das außerdem einen Schritt d) umfasst, in dem man die Mikroorganismen in der Rückhaltezone wachsen lässt.

13. Aufkonzentrierungsverfahren nach einem der Ansprüche 11 und 12, wobei die Mikroorganismen auf den Lysemitteln, die in der Rückhaltezone vorhanden sind, zurückgehalten werden.

14. Verfahren zur Lyse von in der Luft enthaltenen Mikroorganismen, wobei das Verfahren die Schritte umfasst:
a) Platzieren einer Kartusche nach einem der Ansprüche 1 bis 4 im Inneren des Luftsammelmittels, so dass die Rückhaltezone im Inneren der Kartusche mit dem Lufteinlasskanal des Luftsammelmittels in Kommunikation steht,
b) Herbeiführen des Eintritts von Luft in das Innere des Luftsammelmittels durch jedes geeignete Mittel,
c) Aufkonzentrieren der in der Luft enthaltenen Mikroorganismen in der Rückhaltezone der Kartusche,
d) Entnehmen der Kartusche aus dem Luftsammelmittel,
e) Platzieren des Nukleinsäuregewinnungsmittels in der Kartusche,
f) Einbringen einer Flüssigkeit von Interesse in die Kartusche, wodurch die in der Mikroorganismenrückhaltezone der Kartusche befindlichen Lysemittel suspendiert werden, und
g) mechanisches Lysieren der Mikroorganismen, indem das Nukleinsäuregewinnungsmittel im Inneren der Kartusche in Rotation versetzt wird, wobei das Nukleinsäuregewinnungsmittel die Lysemittel, auf denen die Mikroorganismen zurückgehalten sind, mit in Rotation versetzt.

15. Verfahren zur Lyse von in der Luft enthaltenen Mikroorganismen, wobei das Verfahren die Schritte umfasst:
a) Platzieren einer Kartusche nach einem der Ansprüche 1 bis 4 im Inneren des Luftsammelmittels, so dass die Rückhaltezone im Inneren der Kartusche mit dem Lufteinlasskanal des Luftsammelmittels im Kommunikation steht,
b) Herbeiführen des Eintritts von Luft in das Innere des Luftsammelmittels durch jedes geeignete Mittel,
c) Aufkonzentrieren der in der Luft enthaltenen Mikroorganismen in der Rückhaltezone der Kartusche,
d) Entnehmen der Kartusche aus dem Luftsammelmittel,
e) Platzieren des Nukleinsäuregewinnungsmittels in der Kartusche durch Ineinanderstecken,
f) Herbeiführen von Pumpen einer Flüssigkeit von Interesse, die zuvor in der Aufbewahrungszone des Nukleinsäuregewinnungsmittels platziert wurde, wobei das Pumpen mithilfe des Ansaug-/Pumpmittels des Nukleinsäuregewinnungsmittels erhalten wird, wobei die so gepumpte Flüssigkeit den zwischen dem Nukleinsäuregewinnungsmittel und der Kartusche befindlichen Zwischenraum füllt, wodurch die in der Mikroorganismenrückhaltezone der Kartusche befindlichen Lysemittel suspendiert werden, wobei die Lysemittel sich zwischen der vertikalen Innenwand der Kartusche und der vertikalen Außenwand des Nukleinsäuregewinnungsmittels positionieren, und
g) mechanisches Lysieren der Mikroorganismen, indem das Nukleinsäuregewinnungsmittel im Inneren der Kartusche in Rotation versetzt wird, wobei das Nukleinsäuregewinnungsmittel die Lysemittel, auf denen die Mikroorganismen zurückgehalten sind, mit in Rotation versetzt.

16. Verfahren zur Extraktion der Nukleinsäuren von in der Luft enthaltenen Mikroorganismen, wobei das Verfahren die Schritte umfasst:
a) Platzieren einer Kartusche nach einem der Ansprüche 1 bis 4 im Inneren des Luftsammelmittels, so dass die Rückhaltezone im Inneren der Kartusche mit dem Lufteinlasskanal des Luftsammelmittels in Kommunikation steht,
b) Herbeiführen des Eintritts von Luft in das Innere des Luftsammelmittels durch jedes geeignete Mittel,
c) Aufkonzentrieren der in der Luft enthaltenen Mikroorganismen in der Rückhaltezone der Kartusche,
d) Entnehmen der Kartusche aus dem Luftsammelmittel,
e) Platzieren des Nukleinsäuregewinnungsmittels in der Kartusche,
f) Einbringen einer Flüssigkeit von Interesse in die Kartusche, wodurch die in der Mikroorganismenrückhaltezone der Kartusche befindlichen Lysemittel suspendiert werden, und
g) mechanisches Lysieren der Mikroorganismen, indem das Nukleinsäuregewinnungsmittel im Inneren der Kartusche in Rotation versetzt wird, wobei das Nukleinsäuregewinnungsmittel die Lysemittel, auf denen die Mikroorganismen zurückgehalten sind, mit in Rotation versetzt, und
h) Ansaugen der Flüssigkeit von Interesse, die die während der Lyse freigesetzten Nukleinsäuren der Mikroorganismen enthält.

17. Verfahren zur Extraktion von Nukleinsäuren von in der Luft enthaltenen Mikroorganismen, wobei das Verfahren die Schritte umfasst:
a) Platzieren einer Kartusche nach einem der Ansprüche 1 bis 4 im Inneren des Luftsammelmittels, so dass die Rückhaltezone im Inneren der Kartusche mit dem Lufteinlasskanal des Luftsammelmittels in Kommunikation steht,
b) Herbeiführen des Eintritts von Luft in das Innere des Luftsammelmittels durch jedes geeignete Mittel,
c) Aufkonzentrieren der in der Luft enthaltenen Mikroorganismen in der Rückhaltezone der Kartusche,
d) Entnehmen der Kartusche aus dem Luftsammelmittel,
e) Platzieren des Nukleinsäuregewinnungsmittels in der Kartusche durch Ineinanderstecken,
f) Herbeiführen von Pumpen einer Flüssigkeit von Interesse, die zuvor in der Aufbewahrungszone des Nukleinsäuregewinnungsmittels platziert wurde, wobei das Pumpen mithilfe des Ansaug-/Pumpmittels des Nukleinsäuregewinnungsmittels erhalten wird, wobei die so gepumpte Flüssigkeit den zwischen dem Nukleinsäuregewinnungsmittel und der Kartusche befindlichen Zwischenraum füllt, wodurch die in der Mikroorganismenrückhaltezone der Kartusche befindlichen Lysemittel suspendiert werden, wobei die Lysemittel sich zwischen der vertikalen Innenwand der Kartusche und der vertikalen Außenwand des Nukleinsäuregewinnungsmittels positionieren,
g) mechanisches Lysieren der Mikroorganismen, indem das Nukleinsäuregewinnungsmittel im Inneren der Kartusche in Rotation versetzt wird, wobei das Nukleinsäuregewinnungsmittel die Lysemittel, auf denen die Mikroorganismen zurückgehalten sind, mit in Rotation versetzt, wodurch die Nukleinsäuren der Mikroorganismen freigesetzt werden, und
h) Herbeiführen von Ansaugen der Flüssigkeit von Interesse in die Aufbewahrungszone des Nukleinsäuregewinnungsmittels, wobei das Ansaugen mithilfe des Ansaug-/Pumpmittels des Nukleinsäuregewinnungsmittels erhalten wird, wobei die so angesaugte Flüssigkeit von Interesse die während der Lyse freigesetzten Nukleinsäuren der Mikroorganismen enthält.

18. Verfahren nach einem der Ansprüche 14 bis 17, umfassend einen zusätzlichen Schritt d'), in dem man die in der Rückhaltezone der Kartusche aufkonzentrierten Mikroorganismen wachsen lässt.

19. Verfahren nach dem vorhergehenden Anspruch, wobei das Wachsenlassen durch Inkubation der Kartusche in einem Trockenofen für einen Zeitraum von 2 bis 24 Stunden erhalten wird.

20. Verfahren zur Lyse von Mikroorganismen, wobei das Verfahren die Schritte umfasst:
a) Erhalten einer Kartusche nach einem der Ansprüche 1 bis 4, in der Mikroorganismen in der Rückhaltezone aufkonzentriert sind,
b) Platzieren des Nukleinsäuregewinnungsmittels in der Kartusche durch Ineinanderstecken,
c) Herbeiführen von Pumpen einer Flüssigkeit von Interesse, die zuvor in der Aufbewahrungszone des Nukleinsäuregewinnungsmittels platziert wurde, wobei das Pumpen mithilfe des Ansaug-/Pumpmittels des Nukleinsäuregewinnungsmittels erhalten wird, wobei die so gepumpte Flüssigkeit den zwischen dem Nukleinsäuregewinnungsmittel und der Kartusche befindlichen Zwischenraum füllt, wodurch die in der Mikroorganismenrückhaltezone der Kartusche befindlichen Lysemittel suspendiert werden, wobei die Lysemittel sich zwischen der vertikalen Innenwand der Kartusche und der vertikalen Außenwand des Nukleinsäuregewinnungsmittels positionieren, und
d) mechanisches Lysieren der Mikroorganismen, indem das Nukleinsäuregewinnungsmittel im Inneren der Kartusche in Rotation versetzt wird, wobei das Nukleinsäuregewinnungsmittel die Lysemittel, auf denen die Mikroorganismen zurückgehalten sind, mit in Rotation versetzt.

21. Verfahren zur Lyse von Mikroorganismen, wobei das Verfahren die Schritte umfasst:
a) Erhalten einer Kartusche nach einem der Ansprüche 1 bis 4, in der Mikroorganismen in der Rückhaltezone aufkonzentriert sind,
b) Platzieren des Nukleinsäuregewinnungsmittels in der Kartusche,
c) Einleiten einer Flüssigkeit von Interesse in die Kartusche, wodurch die in der Mikroorganismenrückhaltezone der Kartusche befindlichen Lysemittel suspendiert werden, und
d) mechanisches Lysieren der Mikroorganismen, indem das Nukleinsäuregewinnungsmittel im Inneren der Kartusche in Rotation versetzt wird, wobei das Nukleinsäuregewinnungsmittel die Lysemittel, auf denen die Mikroorganismen zurückgehalten sind, mit in Rotation versetzt.

22. Verfahren zur Extraktion von Nukleinsäuren aus Mikroorganismen, wobei das Verfahren die Schritte umfasst:
a) Erhalten einer Kartusche nach einem der Ansprüche 1 bis 4, in der Mikroorganismen in der Rückhaltezone aufkonzentriert sind,
b) Platzieren des Nukleinsäuregewinnungsmittels in der Kartusche durch Ineinanderstecken,
c) Herbeiführen von Pumpen einer Flüssigkeit von Interesse, die zuvor in der Aufbewahrungszone des Nukleinsäuregewinnungsmittels platziert wurde, wobei das Pumpen mithilfe des Ansaug-/Pumpmittels des Nukleinsäuregewinnungsmittels erhalten wird, wobei die so gepumpte Flüssigkeit den zwischen dem Nukleinsäuregewinnungsmittel und der Kartusche befindlichen Zwischenraum füllt, wodurch die in der Mikroorganismenrückhaltezone der Kartusche befindlichen Lysemittel suspendiert werden, wobei die Lysemittel sich zwischen der vertikalen Innenwand der Kartusche und der vertikalen Außenwand des Nukleinsäuregewinnungsmittels positionieren,
d) mechanisches Lysieren der Mikroorganismen, indem das Nukleinsäuregewinnungsmittel im Inneren der Kartusche in Rotation versetzt wird, wobei das Nukleinsäuregewinnungsmittel die Lysemittel, auf denen die Mikroorganismen zurückgehalten sind, mit in Rotation versetzt, wodurch die Nukleinsäuren der Mikroorganismen freigesetzt werden, und
e) Herbeiführen von Ansaugen der Flüssigkeit von Interesse in die Aufbewahrungszone des Nukleinsäuregewinnungsmittels, wobei das Ansaugen mithilfe des Ansaug-/Pumpmittels des Nukleinsäuregewinnungsmittels erhalten wird, wobei die so angesaugte Flüssigkeit von Interesse die während der Lyse freigesetzten Nukleinsäuren der Mikroorganismen enthält.

23. Verfahren zur Extraktion von Nukleinsäuren aus Mikroorganismen, wobei das Verfahren die Schritte umfasst:
a) Erhalten, nach einem der Ansprüche 1 bis 4, einer Kartusche, in der Mikroorganismen in der Rückhaltezone aufkonzentriert sind,
b) Platzieren des Nukleinsäuregewinnungsmittels in der Kartusche,
c) Einleiten einer Flüssigkeit von Interesse in die Kartusche, wodurch die in der Mikroorganismenrückhaltezone der Kartusche befindlichen Lysemittel suspendiert werden, und
d) mechanisches Lysieren der Mikroorganismen, indem das Nukleinsäuregewinnungsmittel im Inneren der Kartusche in Rotation versetzt wird, wobei das Nukleinsäuregewinnungsmittel die Lysemittel, auf denen die Mikroorganismen zurückgehalten sind, mit in Rotation versetzt, und
e) Ansaugen der Flüssigkeit von Interesse, die die während der Lyse freigesetzten Nukleinsäuren der Mikroorganismen enthält.

24. Verfahren zur Lyse von Mikroorganismen, wobei das Verfahren die Schritte umfasst:
a) Einbringen einer flüssigen Probe, die die Mikroorganismen enthält, in eine Kartusche nach einem der Ansprüche 1 bis 4 in die Nähe der Rückhaltezone, so dass die flüssige Probe das Suspendieren der in der Mikroorganismenrückhaltezone des Kartuschen befindlichen Lysemittel bewirkt,
b) Platzieren des Nukleinsäuregewinnungsmittels in der Kartusche,
c) mechanisches Lysieren der Mikroorganismen, indem das Nukleinsäuregewinnungsmittel im Inneren der Kartusche in Rotation versetzt wird, wobei das Nukleinsäuregewinnungsmittel die Lysemittel, auf denen die Mikroorganismen zurückgehalten sind, mit in Rotation versetzt.

25. Verfahren zur Extraktion von Nukleinsäuren aus Mikroorganismen, wobei das Verfahren die Schritte umfasst:
a) Einbringen einer flüssigen Probe, die die Mikroorganismen enthält, in eine Kartusche nach einem der Ansprüche 1 bis 4 in die Nähe der Rückhaltezone, so dass die flüssige Probe das Suspendieren der in der Mikroorganismenrückhaltezone des Kartuschen befindlichen Lysemittel bewirkt,
b) Platzieren des Nukleinsäuregewinnungsmittels in die Kartusche,
c) mechanisches Lysieren der Mikroorganismen, indem das Nukleinsäuregewinnungsmittel im Inneren der Kartusche in Rotation versetzt wird, wobei das Nukleinsäuregewinnungsmittel die Lysemittel, auf denen die Mikroorganismen zurückgehalten sind, mit in Rotation versetzt,
d) Ansaugen der Flüssigkeit von Interesse, die die während der Lyse freigesetzten Nukleinsäuren der Mikroorganismen enthält.

26. Verfahren zur Identifizierung eines oder mehrerer in einer Probennahme enthaltener Mikroorganismen, umfassend die Schritte
- Isolieren der Nukleinsäuren der in der Probennahme enthaltenen Mikroorganismen mithilfe der Vorrichtung nach einem der Ansprüche 5 bis 10,
- Identifizieren des Mikroorganismus bzw. der Mikroorganismen, der bzw. die so isoliert wurde(n).

27. Identifizierungsverfahren nach dem vorhergehenden Anspruch, das ferner einen Zwischenschritt umfasst, in dem man die Nukleinsäuren reinigt.

28. Identifizierungsverfahren nach einem der Ansprüche 26 oder 27, wobei der Identifikationsschritt die Teilschritte umfasst:
- spezifisches Amplifizieren der isolierten Nukleinsäuren,
- Nachweis der so amplifizierten Nukleinsäuren.

29. Identifizierungsverfahren nach einem der Ansprüche 26 bis 28, wobei der Identifizierungsschritt in einer Identifizierungsvorrichtung durchgeführt wird, die in Fluidverbindung mit der Kartusche der Vorrichtung nach einem der Ansprüche 7 bis 10 steht.

30. Identifizierungsverfahren nach dem vorhergehenden Anspruch, wobei die isolierten Nukleinsäuren von dem Nukleinsäuregewinnungsmittel der Vorrichtung nach einem der Ansprüche 7 bis 10 zur Identifizierungsvorrichtung überführt werden.

31. Identifizierungsverfahren nach dem vorhergehenden Anspruch, wobei die Überführung der Nukleinsäuren durch Pumpen der Flüssigkeit von Interesse, die die Nukleinsäuren enthält, wobei die Flüssigkeit von Interesse in der Aufbewahrungszone des Nukleinsäuregewinnungsmittels enthalten ist, mithilfe des Ansaug-/Pumpmittels des Nukleinsäuregewinnungsmittels erhalten wird.

## Claims

1. A cartridge, intended to be positioned inside an air collection means and configured to receive a means for recovering nucleic acids, said cartridge being substantially cylindrical and comprising a microorganism retaining zone, said retaining zone comprising microorganism lysis means, constituted by beads with a gelled material, inert, capable of retaining the microorganisms, of keeping the lysis means in place and of dissolving in the presence of a liquid.

2. The cartridge as claimed in the preceding claim, in which the gelled material is a microorganism culture medium.

3. The cartridge as claimed in one of the preceding claims, also comprising a means for connection of an analyzing device.

4. The cartridge as claimed in one of the preceding claims, in which the diameter of the beads is between 200 and 600 µm.

5. A device for collecting microorganisms contained in the air, said device comprising:
- an air collection means, comprising an upper element comprising an air inlet duct and a lower element comprising an air outlet duct, said upper and lower elements being able to be interlocked with one another such that an airflow can be created inside said air collection means;
- a cartridge as claimed in one of claims 1 to 4, positioned inside said air collection means.

6. The device as claimed in the preceding claim, in which the air collection means is capable of being connected to an air recycling circuit.

7. A device for microorganism lysis, with the aim of isolating the nucleic acids from said microorganisms, said device comprising:
- a cartridge as claimed in one of claims 1 to 4, said cartridge comprising microorganisms placed in the microorganism retaining zone;
- a means for recovering nucleic acids, substantially cylindrical, that can be placed in the cartridge, said recovering means cooperating with the microorganism lysis means in order to lyse said microorganisms and enable release of the nucleic acids.

8. The device as claimed in the preceding claim, in which the means for recovering nucleic acids comprises a means for drawing up/delivering liquid.

9. The device as claimed in either of claims 7 or 8, in which the means for recovering nucleic acids also comprise a liquid storage zone.

10. The device as claimed in one of claims 7 to 9, in which the internal diameter of the cartridge is greater than the external diameter of the means for recovering nucleic acids, such that, when the means for recovering nucleic acids is fitted into the cartridge, the distance separating the internal wall of the cartridge from the external wall of the means for recovering nucleic acids is sufficiently large to allow the lysis means to sit in this interstitial space and sufficiently small for the lysis means to be in contact with one or other of said walls.

11. A method for concentrating microorganisms contained in the air, said method comprising the steps consisting in:
a) placing a cartridge as claimed in one of claims 1 to 4 inside the air collection means, such that the retaining zone, inside said cartridge, is in communication with the air inlet duct of the air collection means,
b) causing air to enter said air collection means by any appropriate means,
c) concentrating the microorganisms contained in the air in the retaining zone of the cartridge.

12. The method of concentration as claimed in the preceding claim, which also comprises a step d) consisting in growing the microorganisms in the retaining zone.

13. The method of concentration as claimed in either of claims 11 and 12, in which the microorganisms are retained on the lysis means present in the retaining zone.

14. A method for the lysis of microorganisms contained in the air, said method comprising the steps consisting in:
a) placing a cartridge as claimed in one of claims 1 to 4 inside the air collection means, such that the retaining zone, inside said cartridge, is in communication with the air inlet duct of the air collection means,
b) causing air to enter said air collection means by any appropriate means,
c) concentrating the microorganisms contained in the air, in the retaining zone of the cartridge,
d) removing the cartridge from the air collection means,
e) placing the means for recovering nucleic acids in the cartridge,
f) introducing a liquid of interest into the cartridge, which leads to the lysis means located in the microorganism retaining zone of the cartridge being placed in suspension, and
g) mechanically lyzing the microorganisms by rotating the means for recovering nucleic acids, inside the cartridge, said means for recovering nucleic acids rotating the lysis means on which the microorganisms are retained.

15. A method for the lysis of microorganisms contained in the air, said method comprising the steps consisting in:
a) placing a cartridge as claimed in one of claims 1 to 4 inside the air collection means, such that the retaining zone, inside said cartridge, is in communication with the air inlet duct of the air collection means,
b) causing air to enter said air collection means by any appropriate means,
c) concentrating the microorganisms contained in the air, in the retaining zone of the cartridge,
d) removing the cartridge from the air collection means,
e) fitting the means for recovering nucleic acids into the cartridge,
f) causing the delivery of a liquid of interest previously placed in the storage zone of the means for recovering nucleic acids, said delivery being obtained by means of the drawing up/delivering means of the means for recovering nucleic acids, the liquid thus delivered filling the interstitial space located between the means for recovering nucleic acids and the cartridge, which leads to the lysis means located in the microorganism retaining zone of the cartridge being placed in suspension, said lysis means coming to be positionned between the vertical internal wall of the cartridge and the vertical external wall of the means for recovering nucleic acids, and
g) mechanically lyzing the microorganisms by rotating the means for recovering nucleic acids, inside the cartridge, said means for recovering nucleic acids rotating the lysis means on which the microorganisms are retained.

16. A method for the extraction of nucleic acids from microorganisms contained in the air, said method comprising the steps consisting in:
a) placing a cartridge as claimed in one of claims 1 to 4 inside the air collection means, such that the retaining zone, inside said cartridge, is in communication with the air inlet duct of the air collection means,
b) causing air to enter said air collection means by any appropriate means,
c) concentrating the microorganisms contained in the air, in the retaining zone of the cartridge,
d) removing the cartridge from the air collection means,
e) placing the means for recovering nucleic acids in the cartridge,
f) introducing a liquid of interest into the cartridge, which leads to the lysis means located in the microorganism retaining zone of the cartridge being placed in suspension, and
g) mechanically lyzing the microorganisms by rotating the means for recovering nucleic acids, inside the cartridge, said means for recovering nucleic acids rotating the lysis means on which the microorganisms are retained, and
h) drawing up the liquid of interest containing the nucleic acids of said microorganisms, released during the lysis.

17. A method for the extraction of nucleic acids from microorganisms contained in the air, said method comprising the steps consisting in:
a) placing a cartridge as claimed in one of claims 1 to 4 inside the air collection means, such that the retaining zone, inside said cartridge, is in communication with the air inlet duct of the air collection means,
b) causing air to enter said air collection means by any appropriate means,
c) concentrating the microorganisms contained in the air, in the retaining zone of the cartridge,
d) removing the cartridge from the air collection means,
e) fitting the means for recovering nucleic acids into the cartridge,
f) causing the delivery of a liquid of interest previously placed in the storage zone of the means for recovering nucleic acids, said delivery being obtained by means of the drawing up/delivering means of the means for recovering nucleic acids, the liquid thus delivered filling the interstitial space located between the means for recovering nucleic acids and the cartridge, which leads to the lysis means located in the microorganism retaining zone of the cartridge being placed in suspension, said lysis means coming to be positionned between the vertical internal wall of the cartridge and the vertical external wall of the means for recovering nucleic acids,
g) mechanically lyzing the microorganisms by rotating the means for recovering nucleic acids, inside the cartridge, said means for recovering nucleic acids rotating the lysis means on which the microorganisms are retained, thus releasing the nucleic acids from said microorganisms, and
h) causing the liquid of interest in the storage zone of the means for recovering nucleic acids to be drawn up, said drawing up being obtained by means of the drawing up/delivering means of the means for recovering nucleic acids, the liquid of interest thus drawn up containing the nucleic acids of said microorganisms, released during the lysis.

18. The method as claimed in one of claims 14 to 17, comprising an additional step (d') consisting in growing the concentrated microorganisms in the retaining zone of the cartridge.

19. The method as claimed in the preceding claim, in which the growing is obtained by incubation of the cartridge in an incubator for a period of time ranging from 2 to 24 hours.

20. A method for the lysis of microorganisms, said method comprising the steps consisting in:
a) obtaining a cartridge, as claimed in one of claims 1 to 4, in which microorganisms are concentrated in the retaining zone,
b) fitting the means for recovering nucleic acids into the cartridge,
c) causing the delivery of a liquid of interest previously placed in the storage zone of the means for recovering nucleic acids, said delivery being obtained by means of the drawing up/delivering means of the means for recovering nucleic acids, the liquid thus delivered filling the interstitial space located between the means for recovering nucleic acids and the cartridge, which leads to the lysis means located in the microorganism retaining zone of the cartridge being placed in suspension, said lysis means coming to be positionned between the vertical internal wall of the cartridge and the vertical external wall of the means for recovering nucleic acids, and
d) mechanically lyzing the microorganisms by rotating the means for recovering nucleic acids, inside the cartridge, said means for recovering nucleic acids rotating the lysis means on which the microorganisms are retained.

21. A method for the lysis of microorganisms, said method comprising the steps consisting in:
a) obtaining a cartridge, as claimed in one of claims 1 to 4, in which microorganisms are concentrated in the retaining zone,
b) placing the means for recovering nucleic acids in the cartridge,
c) introducing a liquid of interest into the cartridge, which leads to the lysis means located in the microorganism retaining zone of the cartridge being placed in suspension, and
d) mechanically lyzing the microorganisms by rotating the means for recovering nucleic acids, inside the cartridge, said means for recovering nucleic acids rotating the lysis means on which the microorganisms are retained.

22. A method for the extraction of nucleic acids from microorganisms, said method comprising the steps consisting in:
a) obtaining a cartridge, as claimed in one of claims 1 to 4, in which microorganisms are concentrated in the retaining zone,
b) fitting the means for recovering nucleic acids into the cartridge,
c) causing the delivery of a liquid of interest previously placed in the storage zone of the means for recovering nucleic acids, said delivery being obtained by means of the drawing up/delivering means of the means for recovering nucleic acids, the liquid thus delivered filling the interstitial space located between the means for recovering nucleic acids and the cartridge, which leads to the lysis means located in the microorganism retaining zone of the cartridge being placed in suspension, said lysis means coming to sit between the vertical internal wall of the cartridge and the vertical external wall of the means for recovering nucleic acids,
d) mechanically lyzing the microorganisms by rotating the means for recovering nucleic acids, inside the cartridge, said means for recovering nucleic acids rotating the lysis means on which the microorganisms are retained, thus releasing the nucleic acids from said microorganisms, and
e) causing the liquid of interest in the storage zone of the means for recovering nucleic acids to be drawn up, said drawing up being obtained by means of the drawing up/delivering means of the means for recovering nucleic acids, the liquid of interest thus drawn up containing the nucleic acids of said microorganisms, released during the lysis.

23. A method for the extraction of nucleic acids from microorganisms, said method comprising the steps consisting in:
a) obtaining, as claimed in one of claims 1 to 4, a cartridge in which microorganisms are concentrated in the retaining zone,
b) placing the means for recovering nucleic acids in the cartridge,
c) introducing a liquid of interest into the cartridge, which leads to the lysis means located in the microorganism retaining zone of the cartridge being placed in suspension, and
d) mechanically lyzing the microorganisms by rotating the means for recovering nucleic acids, inside the cartridge, said means for recovering nucleic acids rotating the lysis means on which the microorganisms are retained, and
e) drawing up the liquid of interest containing the nucleic acids of said microorganisms, released during the lysis.

24. A method for the lysis of microorganisms, said method comprising the steps consisting in:
a) introducing a liquid sample containing said microorganisms into a cartridge as claimed in one of claims 1 to 4, in the vicinity of the retaining zone, such that said liquid sample leads to the lysis means located in said microorganism retaining zone of the cartridge being placed in suspension,
b) placing the means for recovering nucleic acids in the cartridge,
c) mechanically lyzing the microorganisms by rotating the means for recovering nucleic acids, inside the cartridge, said means for recovering nucleic acids rotating the lysis means on which the microorganisms are retained.

25. A method for the extraction of nucleic acids from microorganisms, said method comprising the steps consisting in:
a) introducing a liquid sample containing said microorganisms into a cartridge as claimed in one of claims 1 to 4 in the vicinity of the retaining zone, such that said liquid sample leads to the lysis means located in said microorganism retaining zone of the cartridge being placed in suspension,
b) placing the means for recovering nucleic acids in the cartridge,
c) mechanically lysing the microorganisms by rotating the means for recovering nucleic acids, inside the cartridge, said means for recovering nucleic acids rotating the lysis means on which the microorganisms are retained,
d) drawing up the liquid of interest containing the nucleic acids of said microorganisms, released during the lysis.

26. A method for identifying one or more microorganisms contained in a sample, comprising the steps consisting in:
- isolating the nucleic acids from the microorganisms contained in said sample by means of the device as claimed in one of claims 5 to 10,
- identifying the microorganism(s) thus isolated.

27. The method of identification as claimed in the preceding claim, also comprising an intermediate step consisting in purifying the nucleic acids.

28. The method of identification as claimed in either of claims 26 or 27, in which the identification step comprises the substeps consisting in:
- specifically amplifying the isolated nucleic acids,
- detecting the nucleic acids thus amplified.

29. The method of identification as claimed in one of claims 26 to 28, in which the identification step is carried out in an identification device in fluid communication with the cartridge of the device as claimed in one of claims 7 to 10.

30. The method of identification as claimed in the preceding claim, in which the isolated nucleic acids are transferred from the means for recovering nucleic acids, of the device as claimed in one of claims 7 to 10, to the identification device.

31. The method of identification as claimed in the preceding claim, in which the transfer of the nucleic acids is obtained by delivery of the liquid of interest containing the nucleic acids, said liquid of interest being contained in the storage zone of the means for recovering nucleic acids, by means of the drawing up/delivering means of the means for recovering nucleic acids.
